(19) Europäisches Patentamt
European Patent Office
Office européen des brevets

(11) **EP 4 445 827 A1**

(12) **EUROPEAN PATENT APPLICATION**
published in accordance with Art. 153(4) EPC

(43) Date of publication:
**16.10.2024 Bulletin 2024/42**

(21) Application number: **22918727.3**

(22) Date of filing: **17.11.2022**

(51) International Patent Classification (IPC):
*A61B 1/045* (2006.01)    *A61B 1/00* (2006.01)

(52) Cooperative Patent Classification (CPC):
**A61B 1/00; A61B 1/045**

(86) International application number:
**PCT/JP2022/042708**

(87) International publication number:
**WO 2023/132138 (13.07.2023 Gazette 2023/28)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **07.01.2022 JP 2022001732
18.08.2022 JP 2022130625**

(71) Applicant: **FUJIFILM Corporation
Tokyo 106-8620 (JP)**

(72) Inventor: **SAITO, Takaaki
Ashigarakami-gun, Kanagawa 258-8538 (JP)**

(74) Representative: **Dehns Germany Partnerschaft
mbB
Theresienstraße 6-8
80333 München (DE)**

(54) **PROCESSOR DEVICE, OPERATION METHOD THEREFOR, AND ENDOSCOPE SYSTEM**

(57)    Provided are a processor device capable of adaptively determining a threshold value to be determined in accordance with an oxygen saturation, a method for operating the processor device, and an endoscope system.

In an oxygen saturation mode, an oxygen saturation image is generated that includes a high-oxygen-saturation region in which a color tone of a base image is controlled by a first color tone control method and a low-oxygen-saturation region in which the color tone of the base image is controlled by a second color tone control method different from the first color tone control method. In a threshold value determination mode for determining a threshold value, a threshold value calculation region (83) is displayed on a display, and the threshold value is calculated based on at least oxygen saturations in the threshold value calculation region (83), which are calculated in accordance with a threshold value calculation operation.

FIG. 31

START

SWITCH TO THRESHOLD VALUE DETERMINATION MODE

DISPLAY THRESHOLD VALUE CALCULATION REGION

PLACE NORMAL REGION WITHIN THRESHOLD VALUE CALCULATION REGION

EXECUTE THRESHOLD VALUE CALCULATION OPERATION

CALCULATE THRESHOLD VALUE

SWITCH TO OXYGEN SATURATION MODE

DISPLAY OXYGEN SATURATION IMAGE

END

EP 4 445 827 A1

**Description**

**BACKGROUND OF THE INVENTION**

1. Field of the Invention

The present invention relates to a processor device that calculates the oxygen saturation of an observation target, a method for operating the processor device, and an endoscope system.

2. Description of the Related Art

In recent years, oxygen saturation imaging has been known in the medical field using an endoscope. The oxygen saturation imaging is performed by capturing an image of an observation target illuminated with illumination light including a wavelength range in which the absorption coefficient changes in accordance with a change in the oxygen saturation of blood hemoglobin. Then, the captured image is used to change the color tone in accordance with the oxygen saturation to produce an oxygen saturation image, and the oxygen saturation image is displayed on a display.
However, changing the color tone of the entire screen in accordance with the oxygen saturation makes it difficult to grasp morphological observation for the observation target. Accordingly, in JP2012-139482A, only a hypoxic region with an oxygen saturation less than or equal to a threshold value is changed in color tone (blue tone) in accordance with the oxygen saturation, and a region with an oxygen saturation exceeding the threshold value, such as a normal site, maintains its color tone to achieve both the morphological observation and identification of the hypoxic region for observation.

**SUMMARY OF THE INVENTION**

As in JP2012-139482A described above, to achieve both the morphological observation and identification of the hypoxic region for observation, it is necessary to determine a threshold value for the oxygen saturation to distinguish a color-tone maintaining region in which the color tone is maintained from a color-tone changing region in which the color tone is changed. The threshold value for the oxygen saturation is preferably determined by a value (boundary value) of the oxygen saturation indicating a boundary between the normal site and a hypoxic site; however, the boundary value may vary depending on the dynamics of the organ being observed during observation, individual differences between patients, or the like. For this reason, it is required to adaptively determine the threshold value in consideration of the dynamics during observation, individual differences between patients, or the like.
It is an object of the present invention to provide a processor device capable of adaptively determining a threshold value indicating a boundary between a normal site and a hypoxic site in accordance with an oxygen saturation, a method for operating the processor device, and an endoscope system.
A processor device according to the present invention includes a processor, and the processor is configured to generate a base image; generate an oxygen saturation image in an oxygen saturation mode, the oxygen saturation image including a high-oxygen-saturation region and a low-oxygen-saturation region, the high-oxygen-saturation region being a region in which an oxygen saturation exceeds a threshold value and in which a color tone of the base image is controlled by a first color tone control method, the low-oxygen-saturation region being a region in which the oxygen saturation is less than or equal to the threshold value and in which the color tone of the base image is controlled by a second color tone control method different from the first color tone control method; and in a threshold value determination mode for determining the threshold value, display a threshold value calculation region on a display and calculate the threshold value based on at least oxygen saturations in the threshold value calculation region, the oxygen saturations in the threshold value calculation region being calculated in accordance with a threshold value calculation operation.
Preferably, the processor is configured to calculate the oxygen saturations in the threshold value calculation region at a timing at which the threshold value calculation operation is performed, and calculate the threshold value based on a representative value of the oxygen saturations in the threshold value calculation region. Preferably, the threshold value is calculated based on the representative value of the oxygen saturations in the threshold value calculation region and a correction oxygen saturation. Preferably, the threshold value calculation region includes a normal site.
Preferably, the processor is configured to, in a case of accepting the threshold value calculation operation performed a plurality of times and accepting a confirmation operation performed after the threshold value calculation operation is performed the plurality of times, perform first processing and second processing, the first processing being for calculating the oxygen saturations in the threshold value calculation region as threshold-value-calculation oxygen saturations at a timing at which the threshold value calculation operation is performed, the second processing being for calculating the threshold value in response to the confirmation operation being performed, the threshold value being calculated based on the threshold-value-calculation oxygen saturations calculated in each threshold value calculation operation.

Preferably, the threshold value is calculated based on a correction oxygen saturation and an average value of representative values of the threshold-value-calculation oxygen saturations calculated in respective threshold value calculation operations. Preferably, the threshold value calculation region includes a normal site or a hypoxic site. Preferably, the correction oxygen saturation is determined based on dynamics of an observation target or individual differences between patients.

Preferably, when the first color tone control method is a method for maintaining the color tone of the base image regardless of the oxygen saturation, the second color tone control method is a method for changing the color tone of the base image in accordance with the oxygen saturation, and when the first color tone control method is a method for changing the color tone of the base image in accordance with the oxygen saturation, the second color tone control method is a method for maintaining the color tone of the base image regardless of the oxygen saturation.

An endoscope system according to the present invention includes the processor device according to the present invention described above, and a light source device that emits first illumination light, second illumination light, and third illumination light in a specific range, the first illumination light including a short-wavelength-side wavelength range in which an absorption coefficient changes in accordance with a change in oxygen saturation of blood hemoglobin. The processor is configured to generate the base image based on a second illumination light image that is based on the second illumination light; and calculate the oxygen saturation based on a first illumination light image that is based on the first illumination light, the second illumination light image, and a third illumination light image that is based on the third illumination light.

An endoscope system according to the present invention includes the processor device according to the present invention described above, and a light source device that emits first illumination light and second illumination light, the first illumination light including a long-wavelength-side wavelength range in which an absorption coefficient changes in accordance with a change in oxygen saturation of blood hemoglobin. The processor is configured to generate the base image based on a second illumination light image that is based on the second illumination light; and calculate the oxygen saturation based on a first illumination light image and the second illumination light image, the first illumination light image being based on the first illumination light.

An endoscope system according to the present invention includes the processor device according to the present invention described above, a light source device that supplies white light to an endoscope, and an imaging unit that is to be attached to the endoscope and that disperses the white light from the endoscope into light of a plurality of wavelength ranges and acquires a base-image-generation image signal to be used to generate the base image and an oxygen-saturation-calculation image signal to be used to calculate the oxygen saturation, based on the dispersed light of the plurality of wavelength ranges.

A method for operating a processor device according to the present invention includes the steps of, by a processor, generating a base image; generating an oxygen saturation image in an oxygen saturation mode, the oxygen saturation image including a high-oxygen-saturation region and a low-oxygen-saturation region, the high-oxygen-saturation region being a region in which an oxygen saturation exceeds a threshold value and in which a color tone of the base image is controlled by a first color tone control method, the low-oxygen-saturation region being a region in which the oxygen saturation is less than or equal to the threshold value and in which the color tone of the base image is controlled by a second color tone control method different from the first color tone control method; and in a threshold value determination mode for determining the threshold value, displaying a threshold value calculation region on a display and calculating the threshold value based on at least oxygen saturations in the threshold value calculation region, the oxygen saturations in the threshold value calculation region being calculated in accordance with a threshold value calculation operation.

According to the present invention, it is possible to adaptively determine a threshold value, and therefore it is possible to more accurately discriminate between a normal site and a hypoxic site.

## BRIEF DESCRIPTION OF THE DRAWINGS

Fig. 1 is a schematic diagram of an endoscope system for digestive-tract endoscopy;
Fig. 2 is an explanatory diagram illustrating display styles on a display and an extension display in a normal mode;
Fig. 3 is an explanatory diagram illustrating display styles on the display and the extension display in an oxygen saturation mode;
Fig. 4 is an explanatory diagram illustrating a display style of the extension display presented at a timing of switching to the oxygen saturation mode;
Fig. 5A is an image diagram of the extension display that displays an internal-digestive-tract oxygen saturation image, and Fig. 5B is an image diagram of the extension display that displays a serosa-side oxygen saturation image;

Fig. 6 is a block diagram illustrating functions of an endoscope system according to a first embodiment;

Fig. 7 is a graph illustrating emission spectra of white light;

Figs. 8A, 8B, and 8C are graphs illustrating emission spectra of first illumination light, emission spectra of second illumination light, and an emission spectrum of third illumination light, respectively;

Fig. 9 is a graph illustrating spectral sensitivity of an imaging sensor;

Fig. 10 is a table illustrating illumination and image signals to be acquired in the normal mode;

Fig. 11 is a table illustrating illumination and image signals to be acquired in the oxygen saturation mode or a correction mode;

Fig. 12 is an explanatory diagram illustrating light emission control and display control in the oxygen saturation mode or the correction mode;

Fig. 13 is a graph illustrating reflection spectra of hemoglobin that differ depending on the blood concentration;

Fig. 14 is a graph illustrating reflection spectra of hemoglobin, which differ depending on the concentration of a yellow pigment, and an absorption spectrum of the yellow pigment;

Fig. 15 is a table illustrating oxygen saturation dependence, blood concentration dependence, and brightness dependence of a B 1 image signal, a G2 image signal, and an R2 image signal without the influence of the yellow pigment;

Fig. 16 is a graph illustrating contours representing the oxygen saturation;

Fig. 17 is a table illustrating oxygen saturation dependence, blood concentration dependence, and brightness dependence related to values on an X-axis indicating a signal ratio ln (R2/G2) and values on a Y-axis indicating a signal ratio ln (B 1/G2);

Fig. 18 is a table illustrating oxygen saturation dependence, blood concentration dependence, yellow pigment dependence, and brightness dependence of a B 1 image signal, a G2 image signal, and an R2 image signal with the influence of the yellow pigment;

Fig. 19 is an explanatory diagram illustrating the oxygen saturation in the presence of the yellow pigment and the oxygen saturation in the absence of the yellow pigment when the observation target has the same oxygen saturation;

Fig. 20 is a table illustrating oxygen saturation dependence, blood concentration dependence, yellow pigment dependence, and brightness dependence of a B 1 image signal, a B3 image signal, G2 and G3 image signals, an R2 image signal, and a B2 image signal with the influence of the yellow pigment;

Fig. 21 is a graph illustrating curved surfaces representing the oxygen saturation in accordance with the yellow pigment;

Figs. 22A and 22B are explanatory diagrams of a case where the state of the oxygen saturation represented by three-dimensional coordinates of X, Y, and Z is represented by two-dimensional coordinates of X and Y;

Fig. 23 is a table illustrating oxygen saturation dependence, blood concentration dependence, yellow pigment dependence, and brightness dependence related to values on the X-axis indicating the signal ratio ln (R2/G2), values on the Y-axis indicating the signal ratio ln (B1/G2), and values on a Z-axis indicating a signal ratio ln (B3/G3);

Fig. 24 is a block diagram illustrating functions of an extension processor device;

Fig. 25 is an explanatory diagram illustrating a method for calculating the oxygen saturation;

Fig. 26 is an explanatory diagram illustrating an amount of color tone adjustment that changes in accordance with the oxygen saturation;

Fig. 27 is an explanatory diagram illustrating an amount of color tone adjustment that changes in accordance with the oxygen saturation (different from that in Fig. 26);

Fig. 28 is an explanatory diagram illustrating a method for generating a contour corresponding to a specific pigment concentration;

Fig. 29 is a block diagram illustrating functions of a threshold value determination unit according to the first embodiment;

Fig. 30 is an image diagram of the extension display indicating a threshold value calculation region;

Fig. 31 is a flowchart illustrating the flow of a threshold value determination mode according to the first embodiment;

Fig. 32 is a block diagram illustrating functions of the threshold value determination unit according to a second embodiment;

Fig. 33 is a flowchart illustrating the flow of the threshold value determination mode according to the second embodiment;

Fig. 34 is a block diagram illustrating functions of a rotary-filter-based endoscope system;

Fig. 35 is a plan view of a rotary filter;

Fig. 36 is an explanatory diagram illustrating a difference value $\Delta Z$ to be used in calculation value correction processing;

Fig. 37 is an explanatory diagram illustrating a calculation method of specific oxygen saturation calculation processing;

Fig. 38 is a schematic diagram of an endoscope system for laparoscopic endoscopy;

Fig. 39 is a graph illustrating emission spectra of mixed light;

Fig. 40 is a block diagram illustrating functions of an imaging unit;

Fig. 41 is a graph illustrating emission spectra of violet light and second blue light;

Fig. 42 is a graph illustrating an emission spectrum of first blue light;

Fig. 43 is a graph illustrating an emission spectrum of green light;

Fig. 44 is a graph illustrating an emission spectrum of red light;

Fig. 45 is a graph illustrating a wavelength range Rk in reflection spectra of hemoglobin that differ depending on the concentration of the yellow pigment;

Fig. 46 is a table illustrating oxygen saturation dependence, blood concentration dependence, yellow pigment dependence, and brightness dependence of G2 and G3 image signals, an R2 image signal, and an Rk image signal with the influence of the yellow pigment;

Fig. 47 is an explanatory diagram of a two-sensor laparoscopic endoscope having a camera head having a color imaging sensor and a monochrome imaging sensor;

Figs. 48A and 48B are graphs illustrating light emission patterns for the two-sensor laparoscopic endoscope, in which Fig. 48A illustrates a light emission pattern during a white frame, and Fig. 48B illustrates a light emission pattern during a green frame;

Fig. 49A is a graph illustrating the light emission pattern during the white frame, Fig. 49B is a graph illustrating reflectance of a dichroic mirror, Fig. 49C is a graph illustrating light transmittance of the monochrome imaging sensor, and Fig. 49D is a graph illustrating a pixel value of an image signal output from the monochrome imaging sensor during the white frame;

Fig. 50A is a graph illustrating the light emission pattern during the white frame, Fig. 50B is a graph illustrating the reflectance of the dichroic mirror, Fig. 50C is a graph illustrating light transmittance of the color imaging sensor, and Fig. 50D is a graph illustrating a pixel value of an image signal output from the color imaging sensor during the white frame;

Fig. 51A is a graph illustrating the light emission pattern during the green frame, Fig. 51B is a graph illustrating the reflectance of the dichroic mirror, Fig. 51C is a graph illustrating the light transmittance of the color imaging sensor, Fig. 51D is a graph illustrating a pixel value of an image signal output from B pixels of the color imaging sensor during the green frame, and Fig. 51E is a graph illustrating a pixel value of an image signal output from G pixels of the color imaging sensor during the green frame;

Fig. 52 is a table illustrating image signals to be used in the oxygen saturation mode or the correction mode among image signals obtained in the white frame or the green frame;

Fig. 53 is an explanatory diagram illustrating FPGA processing or PC processing;

Fig. 54 is an explanatory diagram illustrating light emission control and image signal sets when a two-sensor laparoscopic endoscope is used;

Fig. 55 is an explanatory diagram illustrating effective pixel data subjected to effective-pixel determination;

Fig. 56 is an explanatory diagram illustrating ROIs;

Fig. 57 is an explanatory diagram illustrating effective pixel data used in the PC processing;

Fig. 58 is an explanatory diagram illustrating reliability calculation, specific pigment concentration calculation, and specific pigment concentration correlation determination; and

Fig. 59 is a graph illustrating a relationship between a pixel value of a G2 image signal and reliability.

## DESCRIPTION OF THE PREFERRED EMBODIMENTS

First Embodiment

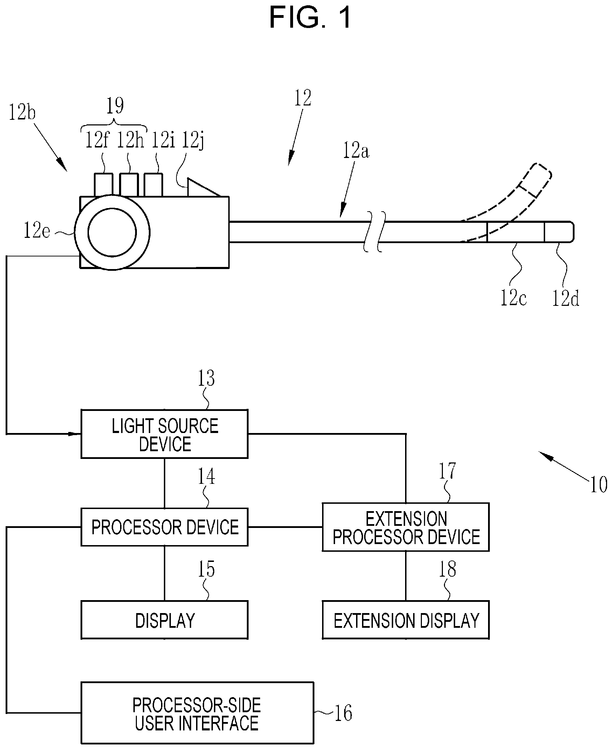

As illustrated in Fig. 1, an endoscope system 10 includes an endoscope 12, a light source device 13, a processor device 14, a display 15, a processor-side user interface 16, an extension processor device 17, and an extension display 18. The endoscope 12 is optically or electrically connected to the light source device 13 and is electrically connected to the processor device 14. The extension processor device 17 is electrically connected to the light source device 13 and the processor device 14. In the claims, a "display" includes the extension display 18 in addition to the display 15.

The endoscope 12 has an insertion section 12a, an operation section 12b, a bending part 12c, and a tip part 12d. The insertion section 12a is inserted into the body of a photographic subject. The operation section 12b is disposed in a proximal end portion of the insertion section 12a. The bending part 12c and the tip part 12d are disposed on the distal end side of the insertion section 12a. The bending part 12c performs a bending operation in response to an operation of an angle knob 12e of the operation section 12b. The tip part 12d is directed in a desired direction by the bending operation of the bending part 12c. A forceps channel (not illustrated) is provided from the insertion section 12a to the tip part 12d to insert a treatment tool or the like through the forceps channel. The treatment tool is inserted into the forceps channel from a forceps port 12j.

The endoscope 12 is internally provided with an optical system for forming a photographic subject image and an optical system for irradiating the photographic subject with illumination light. The operation section 12b is provided with the angle knob 12e, a mode switch 12f, a still-image acquisition instruction switch 12h, and a zoom operation unit 12i. The mode switch 12f is used for an observation mode switching operation. The still-image acquisition instruction switch 12h is used to provide an instruction to acquire a still image of the photographic subject. The zoom operation unit 12i is used to perform an operation of enlarging or shrinking the observation target. The operation section 12b may be provided with the mode switch 12f, the still-image acquisition instruction switch 12h, and a scope-side user interface 19 for performing various operations on the processor device 14.

The light source device 13 generates illumination light. The processor device 14 performs system control of the endoscope system 10 and further performs image processing and the like on an image signal transmitted from the endoscope 12 to generate an endoscopic image, for example. The display 15 displays a medical image transmitted from the processor device 14. The processor-side user interface 16 has a keyboard, a mouse, a microphone, a tablet, a foot switch, a touch pen, and the like, and accepts an input operation such as setting a function.

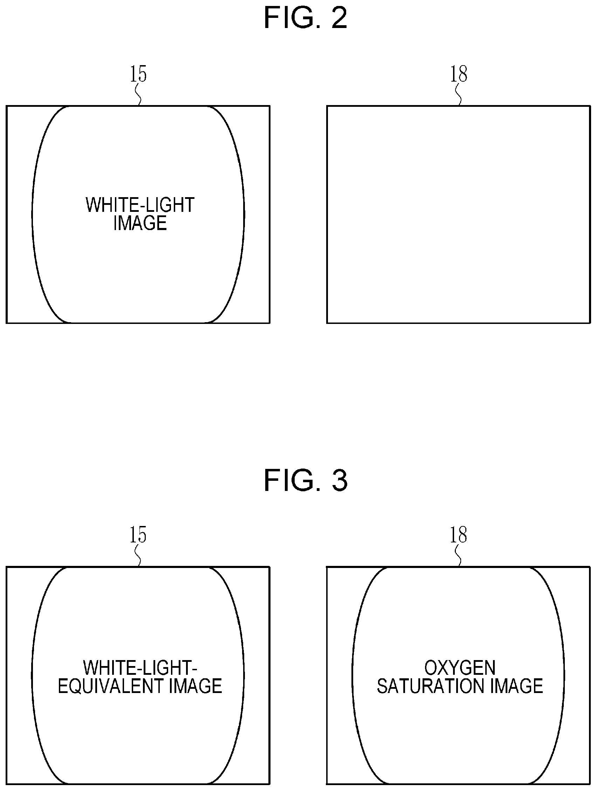

The endoscope system 10 has four modes, namely, a normal mode, an oxygen saturation mode, a correction mode, and a threshold value determination mode, and the four modes are switched by the user operating the mode switch 12f. As illustrated in Fig. 2, in the normal mode, a white-light image with a natural tint, which is obtained by imaging of the observation target using white light as illumination light, is displayed on the display 15, whereas nothing is displayed on the extension display 18.

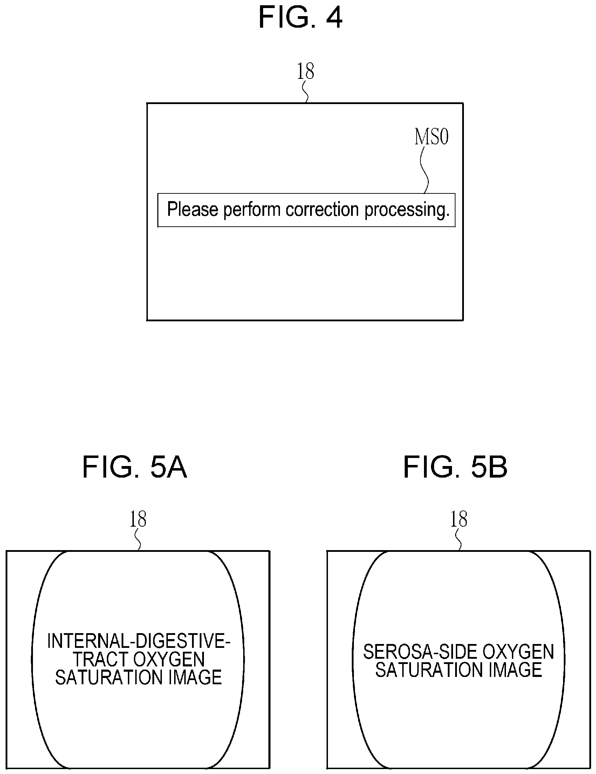

As illustrated in Fig. 3, in the oxygen saturation mode, the oxygen saturation of the observation target is calculated, and an oxygen saturation image that is an image of the calculated oxygen saturation is displayed on the extension display 18. In the oxygen saturation mode, furthermore, a white-light-equivalent image having fewer short-wavelength components than the white-light image is displayed on the display 15. In the correction mode, correction processing related to the calculation of the oxygen saturation is performed on the basis of the specific pigment concentration of a specific pigment other than blood hemoglobin, such as a yellow pigment. When the mode is switched to the oxygen saturation mode, as illustrated in Fig. 4, a message MS0 indicating "Please perform correction processing" is displayed on the extension display 18. When the correction processing is completed, the oxygen saturation image is displayed on the extension display 18. In the threshold value determination mode, a threshold value used to determine a region of the oxygen saturation image in which the color tone corresponding to the oxygen saturation is set is determined.

The endoscope system 10 is of a soft endoscope type for the digestive tract such as the stomach or the large intestine. In the oxygen saturation mode, as illustrated in Fig. 5A, an internal-digestive-tract oxygen saturation image that is an image of the state of the oxygen saturation inside the digestive tract is displayed on the extension display 18. In an endoscope system described below, in the case of a rigid endoscope type for the abdominal cavity such as the serosa, as illustrated in FIG. 5B, an abdominal-cavity-side oxygen saturation image that is an image of the state of the oxygen saturation on the serosa side is displayed on the extension display 18 in the oxygen saturation mode.

In the oxygen saturation mode, it is possible to accurately calculate the oxygen saturation in the following cases:

observation of a predetermined target site (e.g., the esophagus, the stomach, or the large intestine);
environments other than the extracorporeal environment with illumination therearound;
no residue, residual liquid, mucus, blood, or fat remaining on the mucous membrane and the serosa;
no pigment sprayed onto the mucous membrane;
the endoscope 12 located away more than 7 mm from the site to be observed;
observation of the site to be observed with the endoscope at an appropriate distance therebetween without large separation;
a region irradiated with sufficient illumination light;
small specular reflection of light from the site to be observed;
a 2/3 internal region of an oxygen saturation image;
small movement of the endoscope or small movement of the patient such as pulsation or breathing; and
no observation of blood vessels in a deep portion of the mucous membrane of the digestive tract.

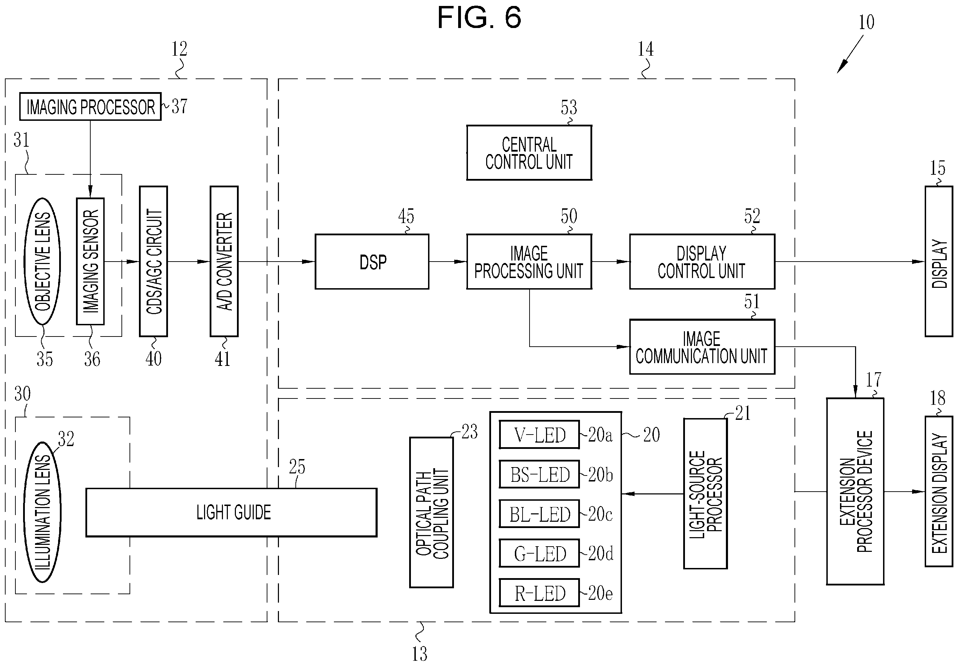

As illustrated in Fig. 6, the light source device 13 includes a light source unit 20 and a light-source processor 21 that controls the light source unit 20. The light source unit 20 has, for example, a plurality of semiconductor light sources and turns on or off each of the semiconductor light sources. The light source unit 20 turns on the semiconductor light sources by controlling the amounts of light to be emitted from the respective semiconductor light sources to emit illumination light for illuminating the observation target. In this embodiment, the light source unit 20 has LEDs of five colors, namely, a V-LED (Violet Light Emitting Diode) 20a, a BS-LED (Blue Short-wavelength Light Emitting Diode) 20b, a BL-LED (Blue Long-wavelength Light Emitting Diode) 20c, a G-LED (Green Light Emitting Diode) 20d, and an R-LED (Red Light Emitting Diode) 20e.

The V-LED 20a emits violet light V of 410 nm $\pm$ 10 nm. The B S-LED 20b emits second blue light BS of 450 nm $\pm$ 10 nm. The BL-LED 20c emits first blue light BL of 470 nm $\pm$ 10 nm. The G-LED 20d emits green light G in the green range. The green light G preferably has a center wavelength of 540 nm. The R-LED 20e emits red light R in the red range. The red light R preferably has a center wavelength of 620 nm. The center wavelengths and the peak wavelengths of the LEDs 20a to 20e may be the same or different.

The light-source processor 21 independently inputs control signals to the respective LEDs 20a to 20e to independently control turning on or off of the respective LEDs 20a to 20e, the amounts of light to be emitted at the time of turning on of the respective LEDs 20a to 20e, and so on. The turn-on or turn-off control performed by the light-source processor 21 differs depending on the mode, which will be described below.

The light emitted from each of the LEDs 20a to 20e is incident on a light guide 25 via an optical path coupling unit 23 constituted by a mirror, a lens, and the like. The light guide 25 is incorporated in the endoscope 12 and a universal cord (a cord that connects the endoscope 12 to the light source device 13 and the processor device 14). The light guide 25 propagates the light from the optical path coupling unit 23 to the tip part 12d of the endoscope 12.

The tip part 12d of the endoscope 12 is provided with an illumination optical system 30 and an imaging optical system 31. The illumination optical system 30 has an illumination lens 32. The illumination light propagating through the light guide 25 is applied to the observation target via the illumination lens 32. The imaging optical system 31 has an objective lens 35 and an imaging sensor 36. Light from the observation target irradiated with the illumination light is incident on the imaging sensor 36 via the objective lens 35. As a result, an image of the observation target is formed on the imaging sensor 36.

The imaging sensor 36 is a color imaging sensor that captures an image of the observation target being illuminated with the illumination light. Each pixel of the imaging sensor 36 is provided with any one of a B pixel (blue pixel) having a B (blue) color filter, a G pixel (green pixel) having a G (green) color filter, and an R pixel (red pixel) having an R (red) color filter. The spectral transmittances of the B color filter, the G color filter, and the R color filter will be described below. For example, the imaging sensor 36 is preferably a color imaging sensor with a Bayer array of B pixels, G pixels, and R pixels, the numbers of which are in the ratio of 1:2:1.

Examples of the imaging sensor 36 can include a CCD (Charge Coupled Device) imaging sensor and a CMOS (Complementary Metal-Oxide Semiconductor) imaging sensor. Instead of the imaging sensor 36 for primary colors, a complementary color imaging sensor including complementary color filters for C (cyan), M (magenta), Y (yellow), and G (green) may be used. When a complementary color imaging sensor is used, image signals of four colors of CMYG are output. Accordingly, the image signals of the four colors of CMYG are converted into image signals of three colors of RGB by complementary-color-to-primary-color conversion. As a result, image signals of the respective colors of RGB similar to those of the imaging sensor 36 can be obtained.

Driving of the imaging sensor 36 is controlled by an imaging processor 37. The control of the respective modes, which is performed by the imaging processor 37, will be described below. A CDS/AGC circuit 40 (Correlated Double Sampling/Automatic Gain Control) performs correlated double sampling (CDS) and automatic gain control (AGC) on an analog image signal obtained from the imaging sensor 36. The image signal having passed through the CDS/AGC circuit 40 is converted into a digital image signal by an A/D converter 41 (Analog/Digital). The digital image signal subjected to A/D conversion is input to the processor device 14.

The processor device 14 includes a DSP (Digital Signal Processor) 45, an image processing unit 50, a display control unit 52, and a central control unit 53. In the processor device 14, programs related to various types of processing are incorporated in a program memory (not illustrated). The central control unit 53, which is constituted by a processor, executes a program in the program memory to implement the functions of the DSP 45, the image processing unit 50, the display control unit 52, and the central control unit 53.

The DSP 45 performs various types of signal processing, such as defect correction processing, offset processing, gain correction processing, linear matrix processing, gamma conversion processing, demosaicing processing, white balance processing, YC conversion processing, and noise reducing processing, on the image signal received from the endoscope 12. In the defect correction processing, a signal of a defective pixel of the imaging sensor 36 is corrected. In the offset processing, a dark current component is removed from the image signal subjected to the defect correction processing, and an accurate zero level is set. The gain correction processing multiplies the image signal of each color after the offset processing by a specific gain to adjust the signal level of each image signal. After the gain correction processing, the image signal of each color is subjected to linear matrix processing for improving color reproducibility.

Thereafter, gamma conversion processing is performed to adjust the brightness and saturation of each image signal. After the linear matrix processing, the image signal is subjected to demosaicing processing (also referred to as isotropic processing or synchronization processing) to generate a signal of a missing color for each pixel by interpolation. Through the demosaicing processing, all the pixels have signals of RGB colors. The DSP 45 performs YC conversion processing on the respective image signals after the demosaicing processing, and outputs brightness signals Y and color difference signals Cb and Cr to the DSP 45. The DSP 45 performs noise reducing processing on the image signals subjected to the demosaicing processing or the like, by using, for example, a moving average method, a median filter method, or the like.

The image processing unit 50 performs various types of image processing on the image signals from the DSP 45. The image processing includes, for example, color conversion processing such as 3 × 3 matrix processing, gradation transformation processing, and three-dimensional LUT (Look Up Table) processing, color enhancement processing, and structure enhancement processing such as spatial frequency enhancement. The image processing unit 50 performs image processing in accordance with the mode. In the normal mode, the image processing unit 50 performs image processing for the normal mode to generate a white-light image. In the oxygen saturation mode, the image processing unit 50 generates a white-light-equivalent image corresponding to a second illumination light image. In the oxygen saturation mode, furthermore, the image processing unit 50 transmits the image signals from the DSP 45 to the extension processor device 17 via an image communication unit 51. Also in the correction mode and the threshold value determination mode, as in the oxygen saturation mode, the image processing unit 50 generates a white-light-equivalent image and transmits the image signals from the DSP 45 to the extension processor device 17 via the image communication unit 51.

The display control unit 52 performs display control for displaying image information such as the white-light image or the oxygen saturation image from the image processing unit 50 and other information on the display 15. In accordance with the display control, the white-light image or the white-light-equivalent image is displayed on the display 15.

The extension processor device 17 receives the image signals from the processor device 14 and performs various types of image processing. In the oxygen saturation mode, the extension processor device 17 calculates the oxygen saturation and generates an oxygen saturation image that is an image of the calculated oxygen saturation. The generated oxygen saturation image is displayed on the extension display 18. In the correction mode, the extension processor device 17 calculates a specific pigment concentration in accordance with a user operation and performs correction processing related to the calculation of the oxygen saturation on the basis of the calculated specific pigment concentration. The details of the oxygen saturation mode and the correction mode performed by the extension processor device 17 will be described below.

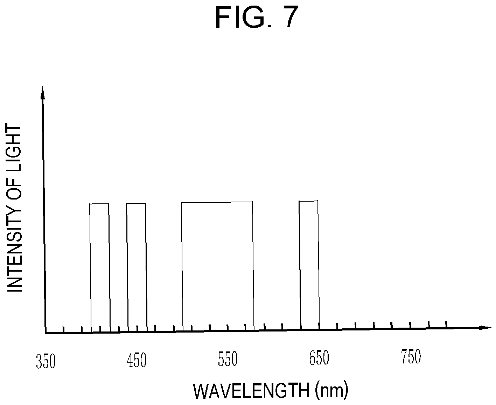

The turn-on or turn-off control in each mode will be described. In the normal mode, when the V-LED 20a, the BS-LED 20b, the G-LED 20d, and the R-LED 20e are simultaneously turned on, as illustrated in Fig. 7, white light including violet light V having a center wavelength of 410 nm, second blue light BS having a center wavelength of 450 nm, broadband green light G in the green range, and red light R having a center wavelength of 620 nm is emitted.

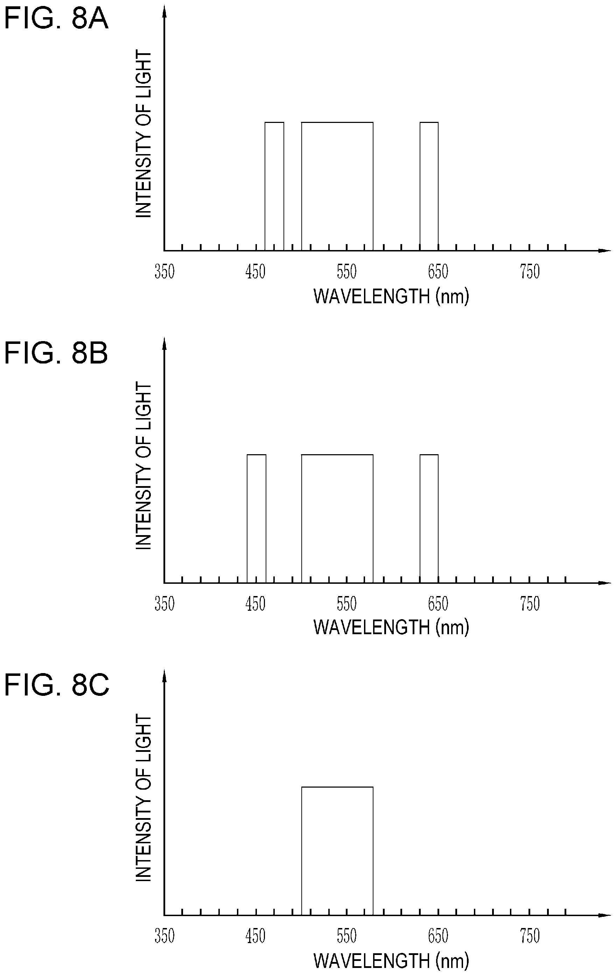

In the oxygen saturation mode, the correction mode, and the threshold value determination mode, light emission for three frames with different light emission patterns is repeatedly performed. In the first frame, as illustrated in Fig. 8A, the BL-LED 20c, the G-LED 20d, and the R-LED 20e are simultaneously turned on to emit broadband first illumination light including first blue light BL having a center wavelength of 470 nm, broadband green light G in the green range, and red light R having a center wavelength of 620 nm. In the second frame, as illustrated in Fig. 8B, the BS-LED 20b, the G-LED 20d, and the R-LED 20e are simultaneously turned on to emit broadband second illumination light including second blue light BS having a center wavelength of 450 nm, broadband green light G in the green range, and red light R having a center wavelength of 620 nm. In the third frame, as illustrated in Fig. 8C, the G-LED 20d is turned on to emit broadband green light G (third illumination light) in the green range. In the oxygen saturation mode, the first frame and the second frame are frames required to obtain an image signal to be required to calculate the oxygen saturation, and thus light may be emitted in only the first frame and the second frame.

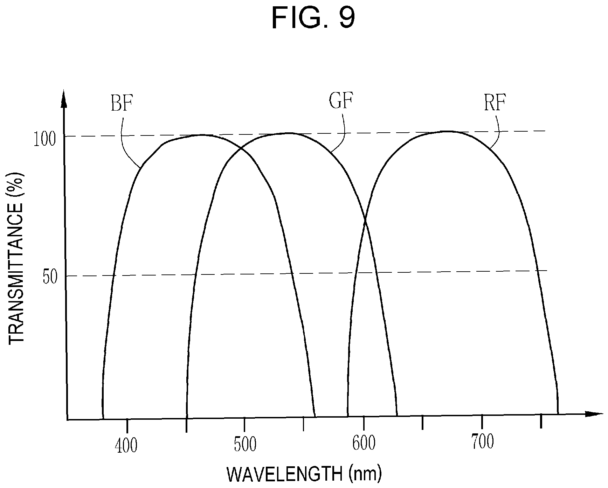

As illustrated in Fig. 9, the B pixels of the imaging sensor 36 are provided with a B color filter BF that mainly transmits light in the blue range, namely, light in the wavelength range of 380 to 560 nm (blue transmission range). A peak wavelength at which the transmittance is maximum appears around 460 to 470 nm. The G pixels of the imaging sensor 36 are provided with a G color filter GF that mainly transmits light in the green range, namely, light in the wavelength range of 450 to 630 nm (green transmission range). The R pixels of the imaging sensor 36 are provided with an R color filter RF that mainly transmits light in the red range, namely, light in the range of 580 to 760 nm (red transmission range).

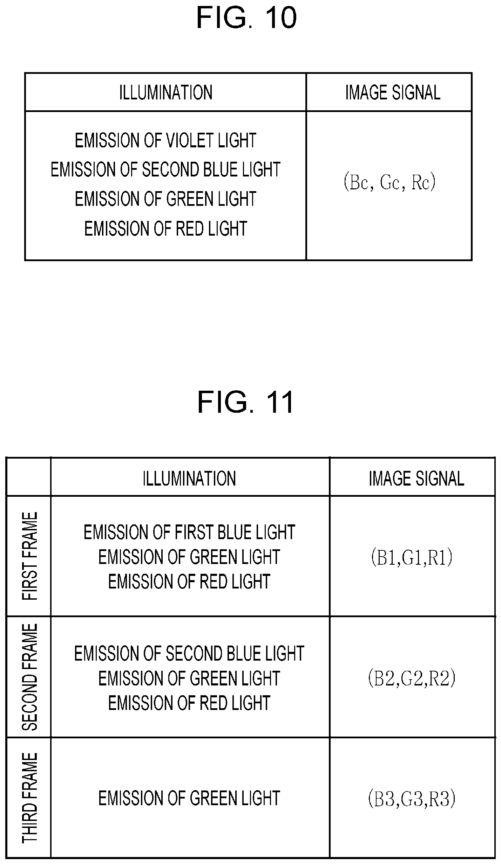

As illustrated in Fig. 10, in the normal mode, the imaging processor 37 controls the imaging sensor 36 to perform imaging of the observation target, which is being illuminated with the violet light V, the second blue light BS, the green light G, and the red light R, frame by frame. As a result, a Bc image signal is output from the B pixels, a Gc image signal is output from the G pixels, and an Rc image signal is output from the R pixels of the imaging sensor 36.

As illustrated in Fig. 11, in the oxygen saturation mode, the correction mode, and the threshold value determination mode, when the observation target is illuminated with the first illumination light including the first blue light BL, the green light G, and the red light R in the first frame, the imaging processor 37 outputs a B 1 image signal from the B pixels, a G1 image signal from the G pixels, and an R1 image signal from the R pixels of the imaging sensor 36 as a first illumination light image. When the observation target is illuminated with the second illumination light including the second blue light BS, the green light G, and the red light R in the second frame, the imaging processor 37 outputs a B2 image signal from the B pixels, a G2 image signal from the G pixels, and an R2 image signal from the R pixels of the imaging sensor 36 as a second illumination light image.

When the observation target is illuminated with the third illumination light that is the green light G in the third frame, the imaging processor 37 outputs a B3 image signal from the B pixels, a G3 image signal from the G pixels, and an R3 image signal from the R pixels of the imaging sensor 36 as a third illumination light image.

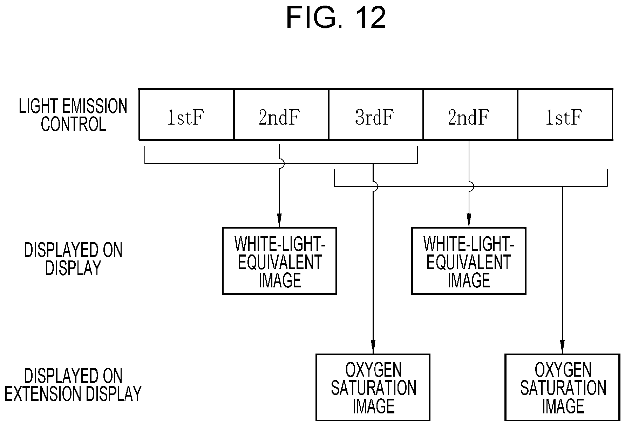

In the oxygen saturation mode, as illustrated in Fig. 12, the first illumination light is emitted in the first frame (1stF), the second illumination light is emitted in the second frame (2ndF), and the third illumination light is emitted in the third frame (3rdF). Thereafter, the second illumination light in the second frame is emitted, and the first illumination light in the first frame is emitted. A white-light-equivalent image obtained on the basis of emission of the second illumination light in the second frame is displayed on the display 15. Further, an oxygen saturation image obtained in response to emission of the first to third illumination light in the first to third frames is displayed on the extension display 18.

In the oxygen saturation mode, of the image signals for the three frames described above, the B 1 image signal included in the first illumination light image, and the G2 image signal and the R2 image signal included in the second illumination light image are used. In the correction mode, to measure the concentration of a specific pigment (such as a yellow pigment) that affects the calculation accuracy of the oxygen saturation, the B3 image signal and the G3 image signal included in the third illumination light image, as well as the B 1 image signal, the G2 image signal, and R2 image signal, are used.

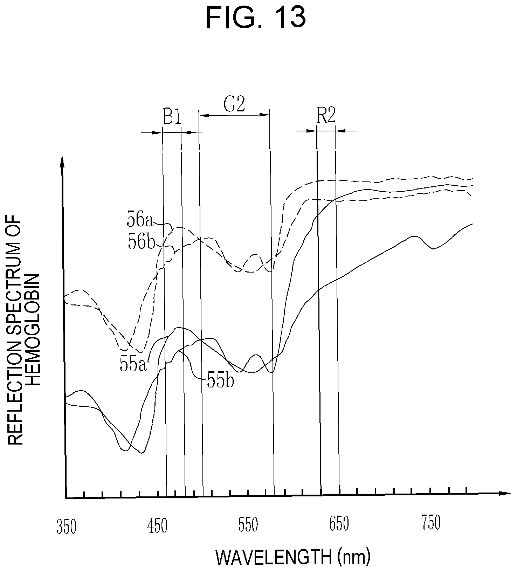

The B 1 image signal includes image information related to at least the first blue light BL of the light transmitted through the B color filter BF out of the first illumination light. The B 1 image signal (oxygen-saturation image signal) includes, as image information related to the first blue light BL, image information of a short-wavelength-side wavelength range B 1 in which the reflection spectrum changes in accordance with a change in the oxygen saturation of blood hemoglobin. As illustrated in Fig. 13, for example, the wavelength range B 1 is preferably a wavelength range from 460 nm to 480 nm including 470 nm at which the difference between the reflection spectra of oxyhemoglobin indicated by curves 55b and 56b and the reflection spectra of reduced hemoglobin indicated by curves 55a and 56a is maximized.

In Fig. 13, the curve 55a represents the reflection spectrum of reduced hemoglobin at a high blood concentration, and the curve 55b represents the reflection spectrum of oxyhemoglobin at a high blood concentration. In contrast, the curve 56a represents the reflection spectrum of reduced hemoglobin at a low blood concentration, and the curve 56b represents the reflection spectrum of oxyhemoglobin at a low blood concentration.

The G2 image signal includes image information of at least a wavelength range G2 related to the green light G of the light transmitted through the G color filter GF out of the first illumination light. For example, as illustrated in Fig. 13, the wavelength range G2 is preferably a wavelength range from 500 nm to 580 nm. The R2 image signal includes image information of at least a wavelength range R2 related to the red light R of the light transmitted through the R color filter RF out of the first illumination light. For example, as illustrated in Fig. 13, the wavelength range R2 is preferably a wavelength range from 610 nm to 630 nm.

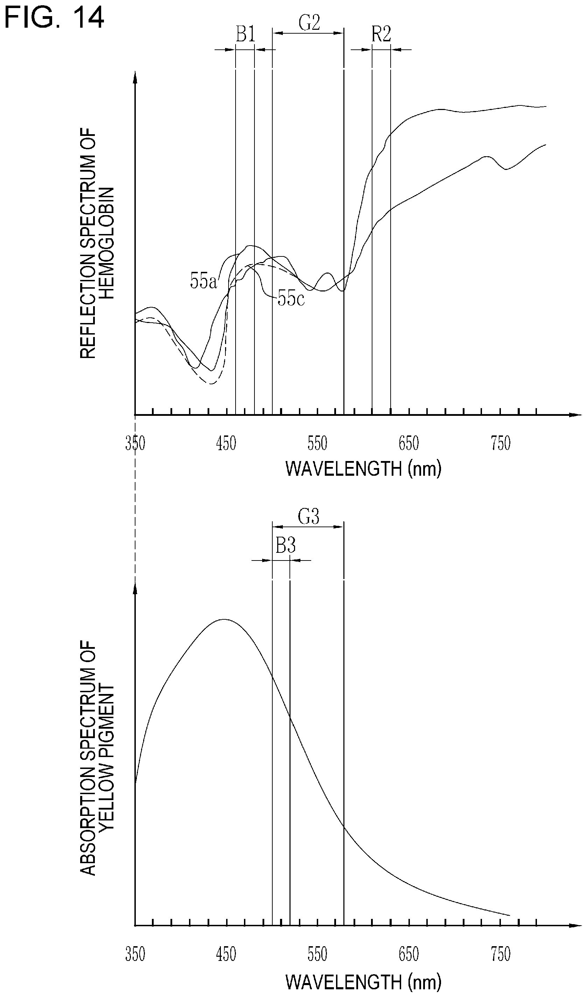

As illustrated in Fig. 14, the image information of the wavelength range B1 includes image information related to the first blue light BL, and the image information of the wavelength range B3 includes image information related to the green light G. The image information related to the first blue light BL and the image information related to the green light G are image information in which the absorption spectrum of a specific pigment such as a yellow pigment changes in accordance with a change in the concentration of the specific pigment. As the absorption spectrum of the specific pigment changes, the reflection spectrum of hemoglobin also changes. The curve 55a represents the reflection spectrum of reduced hemoglobin without the influence of the yellow pigment, and a curve 55c represents the reflection spectrum of reduced hemoglobin with the influence of the yellow pigment. As indicated by the curves 55a and 55c, the reflection spectrum of reduced hemoglobin changes in accordance with the presence or absence of the yellow pigment (the same applies to the reflection spectrum of oxyhemoglobin). Accordingly, in the wavelength range B1 and the wavelength range B3, the reflection spectrum of reduced hemoglobin changes in accordance with a change in the oxygen saturation of blood hemoglobin due to the influence of the specific pigment such as the yellow pigment.

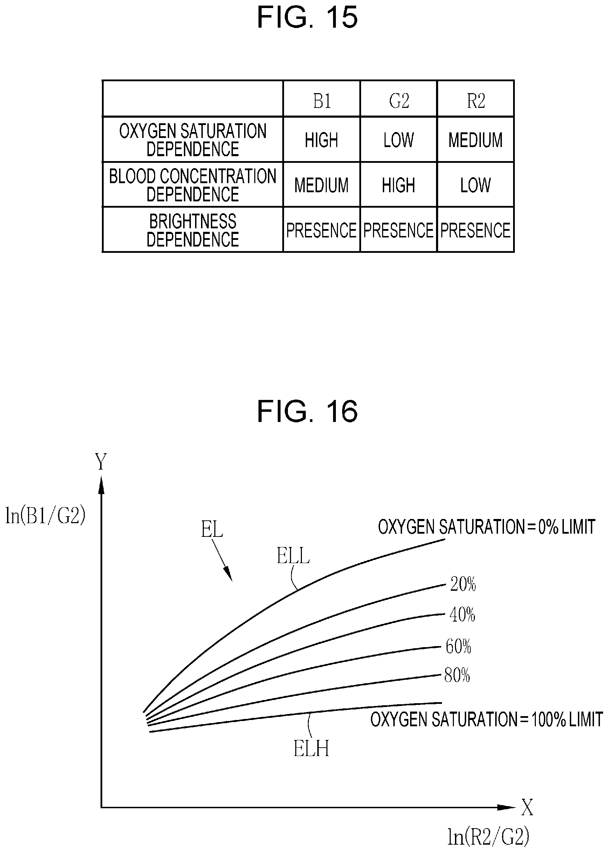

In an ideal case where the observation target is not affected by a specific pigment such as the yellow pigment with the use of the endoscope 12, as illustrated in Fig. 15, the B1 image signal (denoted by "B1"), the G2 image signal (denoted by "G2"), and the R2 image signal (denoted by "R2") are affected by oxygen saturation dependence, blood concentration dependence, or brightness dependence. As described above, since the B1 image signal includes the wavelength range B1 in which the difference between the reflection spectrum of oxyhemoglobin and the reflection spectrum of reduced hemoglobin is maximized, the oxygen saturation dependence, which changes in accordance with the oxygen saturation, is approximately "high". As indicated by the curves 55a and 55b and the curves 56a and 56b, the B1 image signal is approximately "medium" for blood concentration dependence, which changes in accordance with the blood concentration. The B1 image signal has "presence" of brightness dependence, which changes in accordance with the brightness of the observation target. A measure of dependence has "high", "medium", and "low" levels, with the "high" level indicating that the dependence is higher than that of any other image signal, the "medium" level indicating that the dependence is intermediate compared to any other image signal, and the "low" level indicating that the dependence is lower than that of any other image signal.

The G2 image signal has "low" oxygen saturation dependence since the magnitude relationship between the reflection spectrum of oxyhemoglobin and the reflection spectrum of reduced hemoglobin is reversed over a wide wavelength range. As indicated by the curves 55a and 55b and the curves 56a and 56b, the G2 image signal has approximately "high" blood concentration dependence. Like the B1 image signal, the G2 image signal has "presence" of brightness dependence.

The R2 image signal is less likely to be changed by the oxygen saturation than the B1 image signal, but has approximately "medium" oxygen saturation dependence. As indicated by the curves 55a and 55b and the curves 56a and 56b, the R2 image signal has approximately "low" blood concentration dependence. Like the B1 image signal, the G2 image signal has "presence" of brightness dependence.

As described above, since all of the B1 image signal, the G2 image signal, and the R2 image signal have brightness dependence, the G2 image signal is used as a normalized signal to generate an oxygen saturation calculation table 73 for calculating the oxygen saturation by using a signal ratio ln (B1/G2) obtained by normalizing the B1 image signal by the G2 image signal and a signal ratio ln (R2/G2) obtained by normalizing the R2 image signal by the G2 image signal. The term "ln" for the signal ratio ln (B1/G2) is a natural logarithm (the same applies to a signal ratio ln (R2/G2)).

When the relationship between the signal ratios ln (B1/G2) and ln (R2/G2) and the oxygen saturation are represented by two-dimensional coordinates with the signal ratio ln (R2/G2) on the X-axis and the signal ratio ln (B1/G2) on the Y-axis, as illustrated in Fig. 16, the oxygen saturation is represented by contours EL along the Y-axis direction. A contour ELH represents an oxygen saturation of "100%", and a contour ELL represents an oxygen saturation of "0%". The contours are distributed such that the oxygen saturation gradually decreases from the contour ELH to the contour ELL (in Fig. 16, contours for "80%", "60%", "40%", and "20%" are distributed).

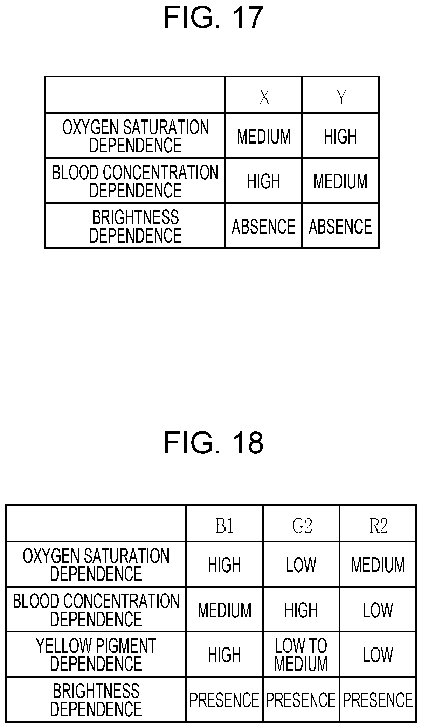

The values (signal ratio ln (R2/G2)) on the X-axis and the values (signal ratio ln (B1/G2)) on the Y-axis are affected by the oxygen saturation dependence and the blood concentration dependence. For the brightness dependence, however, as illustrated in Fig. 17, the values on the X-axis and the values on the Y-axis are normalized by the G2 image signal, and are thus determined to have "absence" without being affected by the brightness dependence. The values on the X-axis have approximately "medium" oxygen saturation dependence and approximately "high" blood concentration dependence. In contrast, the values on the Y-axis have approximately "high" oxygen saturation dependence and approximately "medium" blood concentration dependence.

In an actual case where the observation target is affected by a specific pigment such as the yellow pigment with the use of the endoscope 12, by contrast, as illustrated in Fig. 18, the B1 image signal (denoted by "B1"), the G2 image signal (denoted by "G2"), and the R2 image signal (denoted by "R2") are affected by oxygen saturation dependence, blood concentration dependence, yellow pigment dependence, or brightness dependence. The B1 image signal includes image information in which the absorption spectrum of a specific pigment such as the yellow pigment changes in accordance with a change in the concentration of the specific pigment, and is thus approximately "high" for yellow pigment dependence, which changes in accordance with the yellow pigment. In contrast, the G2 image signal is less likely to be changed by the yellow pigment than the B1 image signal and thus has approximately "low to medium" yellow pigment dependence. The R1 image signal is less likely to be changed by the yellow pigment and thus has approximately "low" yellow pigment dependence.

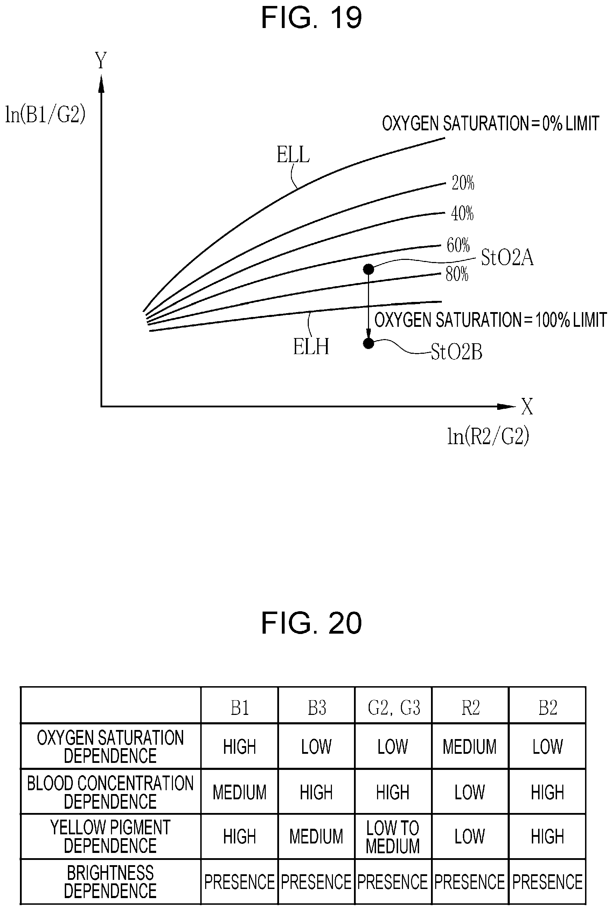

When the signal ratio ln (R2/G2) and the signal ratio ln (B1/G2) are represented by two-dimensional coordinates with the signal ratio ln (R2/G2) on the X-axis and the signal ratio ln (B1/G2) on the Y-axis, even when the observation target has the same oxygen saturation, as illustrated in Fig. 19, an oxygen saturation StO2A in the absence of the yellow pigment and an oxygen saturation StO2b in the presence of the yellow pigment are represented differently. The oxygen saturation of StO2B is apparently shifted to be higher than the oxygen saturation of StO2A due to the presence of the yellow pigment.

Accordingly, for accurate calculation of the oxygen saturation also in the case of yellow pigment dependence, the B3 image signal and the G3 image signal included in the third illumination light image are used to calculate the oxygen saturation. The B3 image signal includes image information related to light transmitted through the B color filter BF out of the third illumination light. The B3 image signal includes image information of the wavelength range B3 having sensitivity to a specific pigment other than hemoglobin, such as the yellow pigment (see Fig. 14). The B3 image signal is less sensitive to the specific pigment than the B1 image signal, but has a certain degree of sensitivity to the specific pigment. Accordingly, as illustrated in Fig. 20, the B1 image signal has "high" yellow pigment dependence, whereas the B3 image signal has approximately "medium" yellow pigment dependence. The B3 image signal has "low" oxygen saturation dependence, "high" blood concentration dependence, and "presence" of brightness dependence.

The G3 image signal also includes an image signal in the wavelength range B3 that is less sensitive to the specific pigment than the G3 image signal but has a certain degree of sensitivity to the specific pigment (see Fig. 14). Accordingly, the G3 image signal has approximately "low to medium" yellow pigment dependence. The G3 image signal has "low" oxygen saturation dependence, "high" blood concentration dependence, and "presence" of brightness dependence. Since the B2 image signal also has "high" yellow pigment dependence, the B2 image signal may be used instead of the B3 image signal to calculate the oxygen saturation. The B2 image signal has "low" oxygen saturation dependence, "high" blood concentration dependence, and "presence" of brightness dependence.

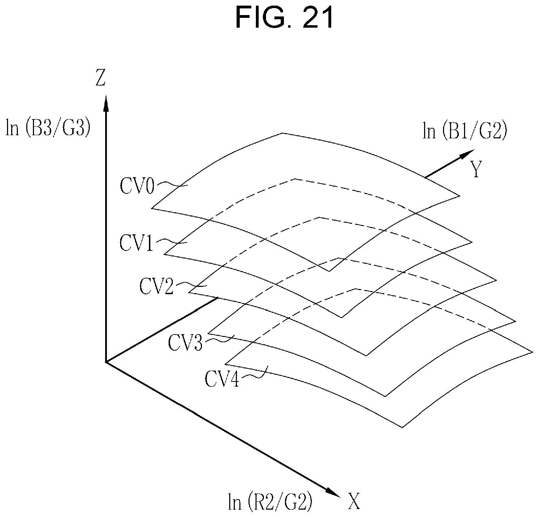

When the relationship between the signal ratios ln (B1/G2) and ln (R2/G2), the yellow pigment, and the oxygen saturation are represented by three-dimensional coordinates with the signal ratio ln (R2/G2) on the X-axis, the signal ratio ln (B1/G2) on the Y-axis, and a signal ratio ln (B3/G3) on the Z-axis, as illustrated in Fig. 21, curved surfaces CV0 to CV4 representing the oxygen saturation are distributed in the Z-axis direction in accordance with the pigment concentration of the yellow pigment. The curved surface CV0 represents the oxygen saturation when the yellow pigment has a concentration of "0" (no influence of the yellow pigment). The curved surfaces CV1 to CV4 represent the oxygen saturations when the yellow pigment has concentrations of "1" to "4", respectively. The concentration having a larger value indicates a higher concentration of the yellow pigment. As indicated by the curved surfaces CV0 to CV4, the values on the Z-axis change so as to decrease as the concentration of the yellow pigment increases.

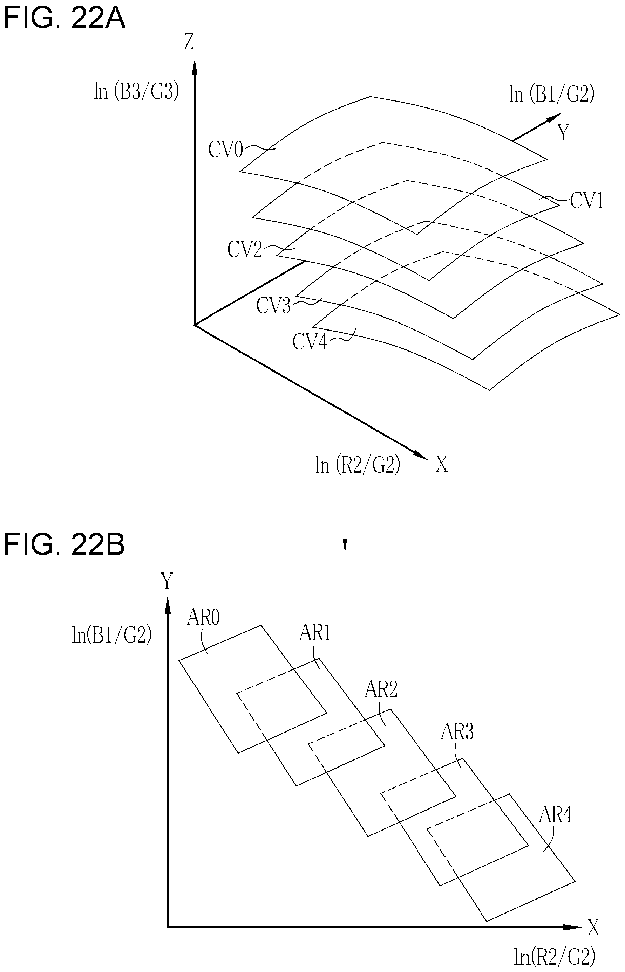

As illustrated in Fig. 22A, when the state of the oxygen saturation represented by three-dimensional coordinates of X, Y, and Z is represented by two-dimensional coordinates of X and Y, as illustrated in Fig. 22B, regions ARC to AR4 representing the respective states of the oxygen saturations are distributed at different positions in accordance with the concentration of the yellow pigment. The regions ARC to AR4 represent the distributions of the oxygen saturations when the yellow pigment has concentrations of "0" to "4", respectively. For each of the regions ARC to AR4, contours EL indicating the oxygen saturations are determined, thereby making it possible to determine an oxygen saturation corresponding to the concentration of the yellow pigment (see Fig. 16). As indicated by the regions ARC to AR4, as the concentration of the yellow pigment increases, the values on the X-axis increase and the values on the Y-axis decrease.

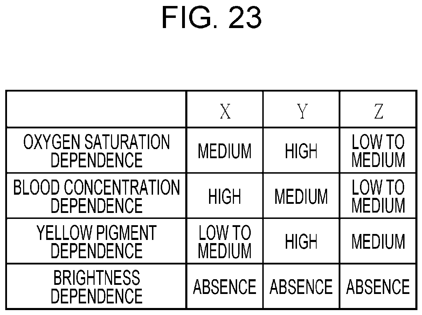

As illustrated in Fig. 23, the values on the X-axis (the signal ratio ln (R2/G2)), the values on the Y-axis (the signal ratio ln (B1/G2)), and the values on the Z-axis (the signal ratio ln (B3/G3)) are subject to yellow pigment dependence. The yellow pigment dependence for the values on the X-axis is "low to medium", the yellow pigment dependence for the values on the Y-axis is "high", and the yellow pigment dependence for the values on the Z-axis is "medium". The values on the Z-axis have "low to medium" oxygen saturation dependence and "low to medium" blood concentration dependence. The values on the Z-axis are normalized by the G3 image signal and thus have "absence" of the brightness dependence.

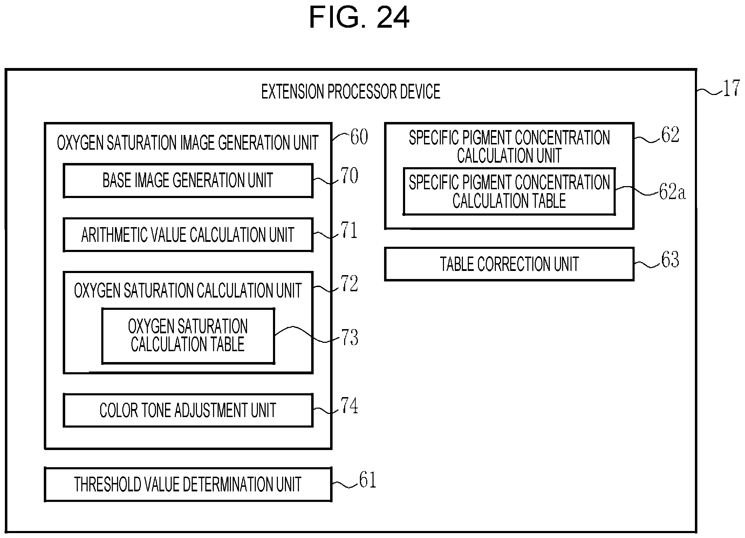

As illustrated in Fig. 24, the extension processor device 17 includes an oxygen saturation image generation unit 60, a threshold value determination unit 61, a specific pigment concentration calculation unit 62, and a table correction unit 63. In the extension processor device 17, programs related to various types of processing are incorporated in a program memory (not illustrated). A central control unit (not illustrated), which is constituted by a processor, executes a program in the program memory to implement the functions of the oxygen saturation image generation unit 60, the threshold value determination unit 61, the specific pigment concentration calculation unit 62, and the table correction unit 63.

The oxygen saturation image generation unit 60 includes a base image generation unit 70, an arithmetic value calculation unit 71, an oxygen saturation calculation unit 72, the oxygen saturation calculation table 73, and a color tone adjustment unit 74. The base image generation unit 70 generates a base image on the basis of the image signals from the processor device 14. The base image is preferably an image from which form information such as the shape of the observation target can be grasped. The base image is constituted by a B2 image signal, a G2 image signal, and an R2 image signal. The base image may be a narrow-band light image in which a blood vessel, a structure (gland duct structure), or the like is highlighted by narrow-band light or the like. In this case, narrow-band light is used as the second illumination light, and imaging of the observation target is performed using the narrow-band light to obtain the narrow-band light image as a second illumination light image.

The arithmetic value calculation unit 71 calculates arithmetic values by arithmetic processing based on the B1 image signal, the G2 image signal, and the R2 image signal included in the oxygen-saturation image signal. Specifically, the arithmetic value calculation unit 71 calculates a signal ratio B1/G2 between the B1 image signal and the G2 image signal and a signal ratio R2/G2 between the R2 image signal and the G2 image signal as arithmetic values to be used for the calculation of the oxygen saturation. The signal ratio B1/G2 and the signal ratio R2/G2 are each preferably converted into a logarithm (ln). Alternatively, color difference signals Cr and Cb, or a saturation S, a hue H, or the like calculated from the B1 image signal, the G2 image signal, and the R2 image signal may be used as the arithmetic values.

The oxygen saturation calculation unit 72 refers to the oxygen saturation calculation table 73 and calculates the oxygen saturation on the basis of the arithmetic values. The oxygen saturation calculation table 73 stores correlations between the signal ratios B1/G2 and R2/G2, each of which is one of the arithmetic values, and the oxygen saturation. When the correlations are represented by two-dimensional coordinates with the signal ratio ln (B1/G2) on the vertical axis and the signal ratio ln (R2/G2) on the horizontal axis, the states of the oxygen saturations are represented by contours EL extending in the horizontal-axis direction, and the contours EL for different oxygen saturations are distributed at different positions in the vertical-axis direction (see Fig. 16).

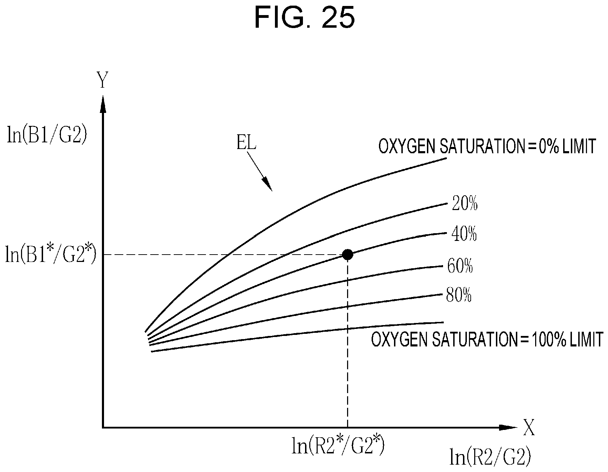

The oxygen saturation calculation unit 72 refers to the oxygen saturation calculation table 73 and calculates, for each pixel, an oxygen saturation corresponding to the signal ratios B1/G2 and R2/G2. For example, as illustrated in Fig. 25, when a specific pixel has signal ratios ln (B1*/G2*) and ln (R2*/G2*), an oxygen saturation corresponding to the signal ratios ln (B1*/G2*) and ln (R2*/G2*) is "40%". Accordingly, the oxygen saturation calculation unit 72 calculates the oxygen saturation of the specific pixel as "40%".

The color tone adjustment unit 74 controls the color tone of the base image in accordance with the oxygen saturation calculated by the oxygen saturation calculation unit 72 to generate an oxygen saturation image. The oxygen saturation image includes a high-oxygen-saturation region that is a region in which the oxygen saturation exceeds a threshold value and in which the color tone of the base image is controlled by a first color tone control method, and a low-oxygen-saturation region that is a region in which the oxygen saturation is less than or equal to the threshold value and in which the color tone of the base image is controlled by a second color tone control method different from the first color tone control method.

In this embodiment, the first color tone control method is a method for maintaining the color tone of the base image regardless of the oxygen saturation, and the second color tone control method is a method for changing the color tone of the base image in accordance with the oxygen saturation. The use of the first color tone control method and the second color tone control method described above makes it possible to maintain the color tone of a normal site in which the oxygen saturation is higher than the threshold value, such as the high-oxygen-saturation region, and to change only the color tone of an abnormal site in which the oxygen saturation is lower than or equal to the threshold value, such as the low-oxygen-saturation region. As a result, the oxygen state of the abnormal site can be grasped in a situation in which morphology information of the normal site can be observed. When the first color tone control method is a method for changing the color tone of the base image in accordance with the oxygen saturation, the second color tone control method is preferably a method for maintaining the color tone of the base image regardless of the oxygen saturation.

As described above, the color tone adjustment unit 74 uses an amount of color tone adjustment CBb for blue by which the B1 image signal is multiplied, an amount of color tone adjustment CBg for green by which the G1 image signal is multiplied, and an amount of color tone adjustment CBr by which the R1 image signal is multiplied to adjust the color tone of the base image. The amounts of color tone adjustment CBb, CBb, and CBr are represented by values larger than 0.

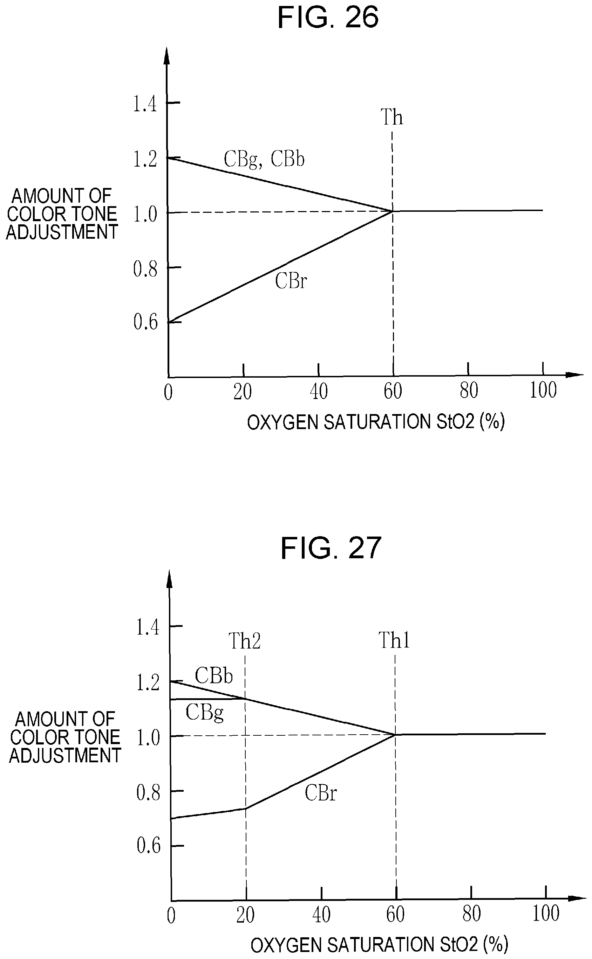

For example, as illustrated in Fig. 26, when a threshold value Th is 60%, the amounts of color tone adjustment CBb, CBg, and CBr are set to "1" for a high-oxygen-saturation region exceeding the threshold value Th in the base image to maintain the color tone. For a low-oxygen-saturation region less than or equal to the threshold value Th in the base image, in contrast, the amounts of color tone adjustment CBb and CBg are set to be larger than "1" and further set to increase as the oxygen saturation decreases to change the color tone in accordance with the oxygen saturation. Further, the amount of color tone adjustment CBr is set to be smaller than "1" and further set to decrease as the oxygen saturation decreases.

The threshold value Th is determined in the threshold value determination mode described below. Not a single threshold value but a plurality of threshold values may be set. For example, as illustrated in Fig. 27, a threshold value Th1 for an oxygen saturation of 60% and a threshold value Th2 for an oxygen saturation of 20% may be set. In this case, similar conditions to those in Fig. 26 are provided when the oxygen saturation is higher than Th1. In a region in which the oxygen saturation is lower than the threshold value Th2, however, the amount of color tone adjustment CBg and the amount of color tone adjustment CBb, which change in accordance with a decrease in the oxygen saturation, change in different amounts. The change in the amount of color tone adjustment CBr, which changes in accordance with a decrease in the oxygen saturation, is smaller than that when the oxygen saturation is higher than the threshold value Th2. Even when a plurality of threshold values are set, it is preferable to determine a relationship (e.g., Th1:Th2 = 3:1 or the like) between the plurality of threshold values in advance and to use one threshold value calculated by a threshold value calculation unit 81 to calculate the other threshold values. When the threshold values Th1 and Th2 are used, a region with an oxygen saturation higher than the threshold value Th1 is a high-oxygen-saturation region, and a region with an oxygen saturation lower than or equal to the threshold value Th1 is a low-oxygen-saturation region.

In the correction mode, the specific pigment concentration calculation unit 62 calculates a specific pigment concentration on the basis of a specific pigment image signal including image information of a wavelength range having sensitivity to a specific pigment other than blood hemoglobin among pigments included in the observation target. Examples of the specific pigment include a yellow pigment such as bilirubin. The specific pigment image signal preferably includes at least the B3 image signal. Specifically, the specific pigment concentration calculation unit 62 calculates the signal ratios ln (B1/G2), ln (R2/G2), and ln (B3/G3). Then, the specific pigment concentration calculation unit 62 refers to a specific pigment concentration calculation table 62a to calculate specific pigment concentrations corresponding to the signal ratios ln (B1/G2), ln (R2/G2), and ln (B3/G3).

The specific pigment concentration calculation table 62a stores correlations between the signal ratios ln (B1/G2), ln (R2/G2), and ln (B3/G3) and the specific pigment concentrations. For example, the range of the signal ratios ln (B1/G2), ln (R2/G2), and ln (B3/G3) is divided into five stages. In this case, the specific pigment concentrations "0" to "4" are stored in the specific pigment concentration calculation table 62a in association with the signal ratios ln (B1/G2), ln (R2/G2), and ln (B3/G3) in the ranges in the five stages, respectively. The signal ratio B3/G3 is preferably converted into a logarithm (ln).

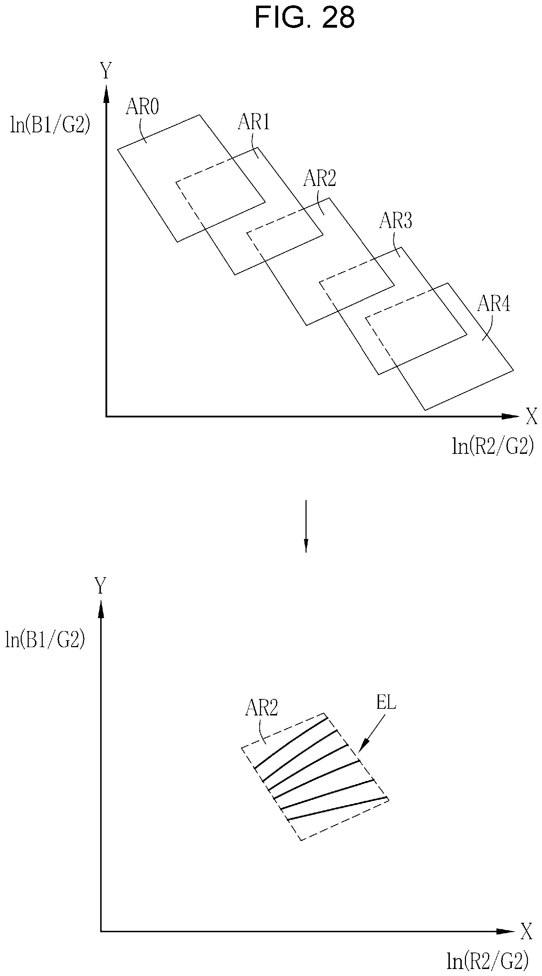

The table correction unit 63 performs, as the correction processing to be performed in the correction mode, table correction processing for correcting the oxygen saturation calculation table 73 on the basis of the specific pigment concentration. The table correction processing corrects the correlations between the signal ratios B1/G2 and R2/G2 and the oxygen saturations, which are stored in the oxygen saturation calculation table 73. Specifically, for the specific pigment concentration "2", as illustrated in Fig. 28, the table correction unit 63 generates contours EL indicating the states of the oxygen saturations in a region AR2 corresponding to the specific pigment concentration "2" among regions ARC to AR4 determined in accordance with the specific pigment concentrations. The table correction unit 63 corrects the oxygen saturation calculation table 73 so as to obtain the generated contours EL.

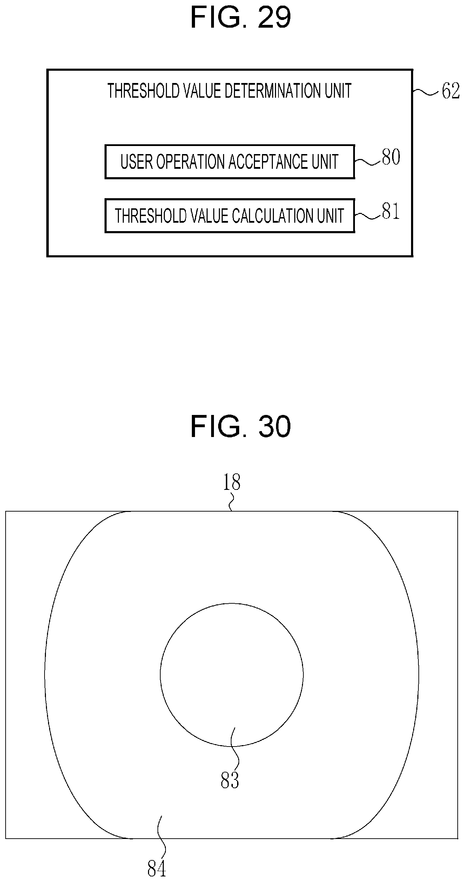

The threshold value determination mode will be described in detail below. The threshold value determination mode is preferably performed before the oxygen saturation mode is executed. However, the threshold value determination mode may be performed, as appropriate, during the execution of the oxygen saturation mode. As illustrated in Fig. 29, the threshold value determination unit 61 includes a user operation acceptance unit 80 and the threshold value calculation unit 81.

The user operation acceptance unit 80 accepts a threshold value calculation operation. The threshold value calculation operation is an operation used to calculate a threshold value to be used for color tone adjustment of an oxygen saturation image. The threshold value calculation operation is performed by the user operating the processor-side user interface 16 or the scope-side user interface 19. When the mode is switched to the threshold value determination mode, the user operation acceptance unit 80 enters a state of being capable of accepting a threshold value calculation operation performed by the processor-side user interface 16 or the scope-side user interface 19.

When the mode is switched to the threshold value determination mode, as illustrated in Fig. 30, the extension display 18 displays a threshold value calculation region 83 in the oxygen saturation image. The threshold value calculation region 83 has a specific shape such as a circular shape. The threshold value calculation region 83 is displayed at a specific position such as the center of the screen in an observation image display region 84 on the display 15. In the threshold value determination mode, unlike the oxygen saturation mode, the observation image display region 84 preferably displays an image such as a normal image in which a normal site (such as a normal mucous membrane) and an abnormal site (such as a hypoxic site) are easily recognized. In this embodiment, in the threshold value determination mode, a normal image constituted by the B1 image signal, the G1 image signal, and the R1 image signal is displayed in the observation image display region 84.

In the first embodiment, in the threshold value determination mode, the user operates the tip part 12d of the endoscope so that a normal site falls within the threshold value calculation region 83. At a timing at which a normal site falls within the threshold value calculation region 83, the user performs a threshold value calculation operation by using the processor-side user interface 16 or the scope-side user interface 19. Accordingly, the threshold value calculation unit 81 calculates oxygen saturations in the threshold value calculation region 83 at a timing at which the threshold value calculation operation is performed. The calculation of the oxygen saturations in the threshold value calculation region 83 is performed by the oxygen saturation calculation unit 72 on the basis of an image (calculation of arithmetic values and the like) of the inside of the threshold value calculation region 83 by using a method similar to that in the oxygen saturation mode.

The threshold value calculation unit 81 calculates the threshold value Th on the basis of the oxygen saturations in the threshold value calculation region 83. The threshold value Th is calculated by using (Equation 1) below on the basis of a representative value RP of the oxygen saturations in the threshold value calculation region 83 and a correction oxygen saturation C. The threshold value Th calculated by the threshold value calculation unit 81 is used for color tone adjustment by the color tone adjustment unit 74. Preferably, the mode is switched to the oxygen saturation mode automatically upon completion of the calculation of the threshold value Th.

Threshold value Th= representative value RP of oxygen saturations in threshold value calculation region 83 - correction oxygen saturation C

In (Equation 1), the representative value RP of the oxygen saturations in the threshold value calculation region 83 is preferably the average value, the median value, the maximum value, or the like. The correction oxygen saturation C is a fixed value determined on the basis of the dynamics of the observation target such as an organ to be observed and individual differences between patients. The use of the correction oxygen saturation C for calculation of the threshold value Th ensures that the threshold value Th is adapted to the dynamics of the observation target or individual differences between patients. (Equation 1) involves subtraction of the correction oxygen saturation C. Any other arithmetic method such as addition may be involved. The correction oxygen saturation C is preferably input in advance by operating the processor-side user interface 16, for example, before endoscopic diagnosis.

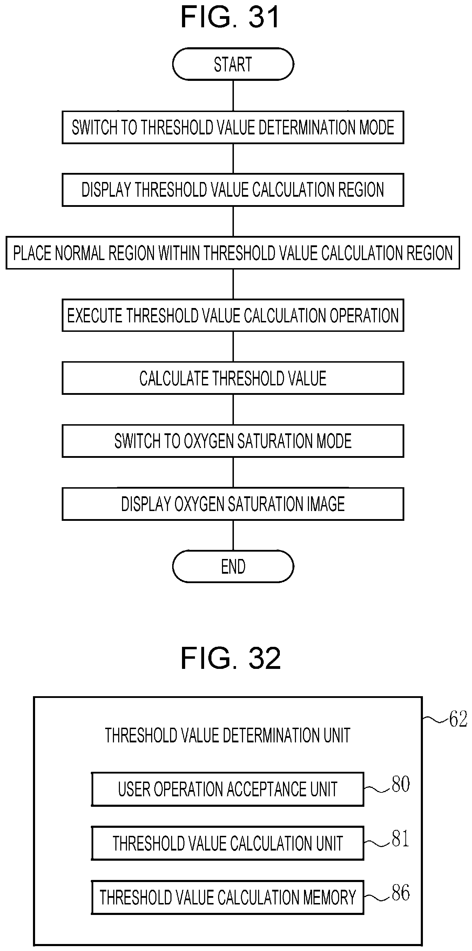

Next, the flow of a series of operations in the threshold value determination mode according to the first embodiment will be described with reference to a flowchart in Fig. 31. A user who desires to display an oxygen saturation image of the observation target operates the mode switch 12f to switch to the threshold value determination mode. Accordingly, the display 15 displays the threshold value calculation region 83.

The user operates the tip part 12d of the endoscope so that a normal site falls within the threshold value calculation region 83. At a timing at which a normal site falls within the threshold value calculation region 83, the user performs a threshold value calculation operation by using the processor-side user interface 16 or the scope-side user interface 19. Accordingly, the threshold value calculation unit 81 calculates oxygen saturations in the threshold value calculation region 83 at a timing at which the threshold value calculation operation is performed. The threshold value calculation unit 81 calculates the threshold value Th on the basis of the oxygen saturations in the threshold value calculation region 83.

When the calculation of the threshold value Th is completed, the threshold value determination mode ends and is automatically switched to the oxygen saturation mode. In the oxygen saturation mode, when an oxygen saturation image is to be displayed on the display 15, the color tone is maintained for a region of the base image where the oxygen saturation exceeds the threshold value, and the color tone is changed to a color tone that changes in accordance with the oxygen saturation for a region of the base image where the oxygen saturation is less than or equal to the threshold value.

Second Embodiment

In the first embodiment, in the threshold value determination mode, a threshold value calculation operation is performed once to determine a threshold value. In a second embodiment, a threshold value calculation operation is performed a plurality of times and then a confirmation operation is performed to determine a threshold value.

In the second embodiment, the user operation acceptance unit 80 accepts the threshold value calculation operation performed a plurality of times and accepts the confirmation operation in accordance with the operation of the processor-side user interface 16 or the scope-side user interface 19. The threshold value calculation unit 81 performs first processing to calculate the oxygen saturations in the threshold value calculation region 83 as threshold-value-calculation oxygen saturations at a timing at which the threshold value calculation operation is performed. As illustrated in Fig. 32, each of the threshold-value-calculation oxygen saturations is stored in a threshold value calculation memory 86 when calculated. In the second embodiment, when the observation target includes an abnormal site such as a hypoxic site, the threshold value calculation operation is preferably performed a plurality of times such that the threshold value calculation region 83 include not only a normal site but also the hypoxic site to determine a threshold value adapted to the observation target.

When the confirmation operation is performed, the threshold value calculation unit 81 performs second processing to calculate a threshold value on the basis of the threshold-value-calculation oxygen saturations calculated in each threshold value calculation operation. The threshold value Th is calculated in accordance with (Equation 2) below on the basis of a plurality-of-operation representative value MRP obtained from representative values RP (e.g., an average value Ave (RP) of representative values RP) of the threshold-value-calculation oxygen saturations, which are calculated in the respective threshold value calculation operations, and the correction oxygen saturation C. As in the first embodiment, the threshold value Th calculated by the threshold value calculation unit 81 is used for color tone adjustment by the color tone adjustment unit 74.

Threshold value Th = plurality-of-operation representative value MRP obtained from representative values of oxygen saturations in threshold value calculation region 83 - correction oxygen saturation C

In (Equation 2), the representative values RP of the threshold-value-calculation oxygen saturations are each preferably the average value, the median value, the maximum value, or the like. The correction oxygen saturation C is similar to that in the first embodiment.

In the calculation of a threshold value in the second embodiment, the threshold-value-calculation oxygen saturations, which are stored in the threshold value calculation memory 86, are read as the threshold-value-calculation oxygen saturations calculated in each threshold value calculation operation and are used to calculate a threshold value. When the mode switch 12f included in the scope-side user interface 19 is used to perform the confirmation operation, for example, the mode switch 12f may be operated twice in succession to perform the confirmation operation.

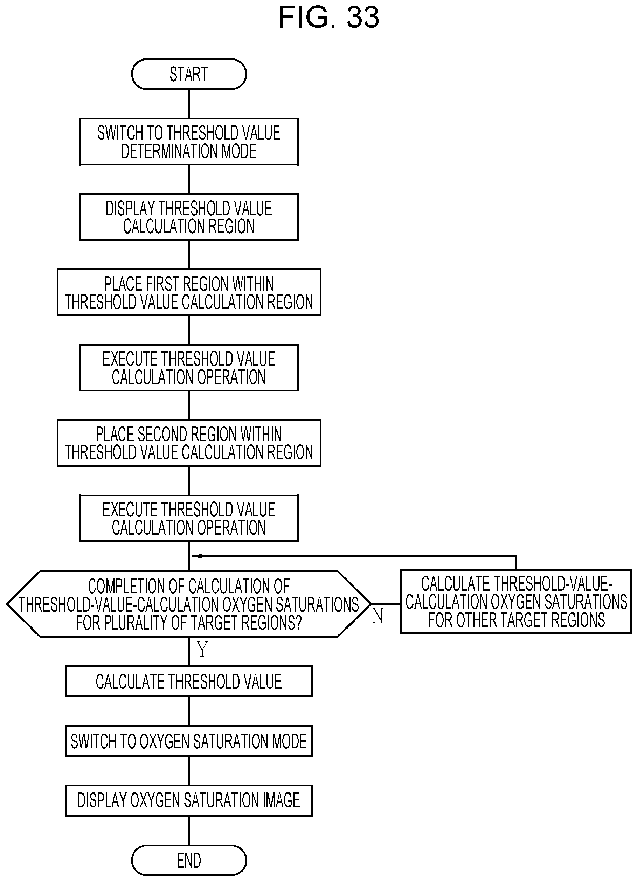

Next, the flow of a series of operations in the threshold value determination mode according to the second embodiment will be described with reference to a flowchart in Fig. 33. The user operates the mode switch 12f to switch to the threshold value determination mode. Accordingly, the display 15 displays the threshold value calculation region 83. In the second embodiment, a threshold value calculation operation is performed a plurality of times to calculate threshold-value-calculation oxygen saturations for a plurality of target regions including a first region and a second region.

The user operates the tip part 12d of the endoscope so that the first region (e.g., a normal site) falls within the threshold value calculation region 83. At a timing at which the first region falls within the threshold value calculation region 83, the user performs a threshold value calculation operation by using the processor-side user interface 16 or the scope-side user interface 19. Accordingly, the threshold value calculation unit 81 calculates the threshold-value-calculation oxygen saturations in the threshold value calculation region 83 at a timing when the threshold value calculation operation is performed. In this case, it is preferable to provide a guidance notification for notifying the user of the completion of the calculation of the threshold-value-calculation oxygen saturations for the first region (the same applies to the completion of the calculation of the threshold-value-calculation oxygen saturations for the second region).

Then, the user operates the tip part 12d of the endoscope so that the second region (e.g., a hypoxic site) falls within the threshold value calculation region 83. At a timing at which the second region falls within the threshold value calculation region 83, the user performs a threshold value calculation operation by using the processor-side user interface 16 or the scope-side user interface 19. Accordingly, the threshold value calculation unit 81 calculates the threshold-value-calculation oxygen saturations in the threshold value calculation region 83 at a timing when the threshold value calculation operation is performed.

When the calculation of the threshold-value-calculation oxygen saturations is completed for the plurality of target regions, the threshold value calculation unit 81 calculates the threshold value Th on the basis of the oxygen saturations in the threshold value calculation region 83, which are calculated in each threshold value calculation operation. When the calculation of the threshold value Th is completed, the threshold value determination mode ends and is automatically switched to the oxygen saturation mode. The color tone adjustment of the oxygen saturations in the oxygen saturation mode is similar to that in the first embodiment.

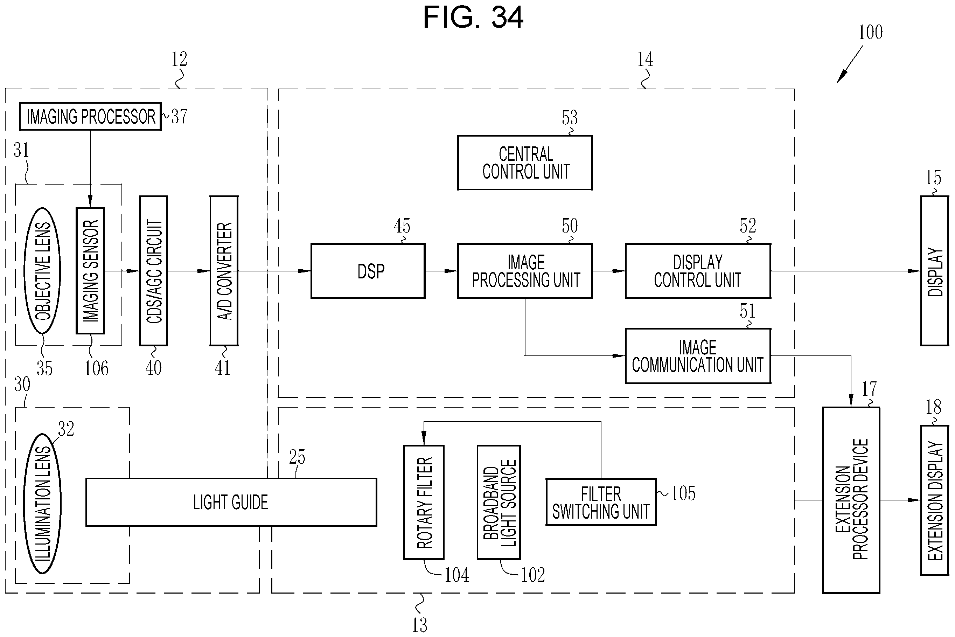

In place of the LEDs 20a to 20e described in the first and second embodiments, a broadband light source such as a xenon lamp and a rotary filter may be used to illuminate the observation target. In this case, as illustrated in Fig. 34, in an endoscope system 100, the light source device 13 is provided with a broadband light source 102, a rotary filter 104, and a filter switching unit 105 in place of the LEDs 20a to 20e. The imaging optical system 31 is provided with, in place of the color imaging sensor 36, a monochrome imaging sensor 106 without a color filter. The other elements are similar to those of the endoscope system 10 described above.

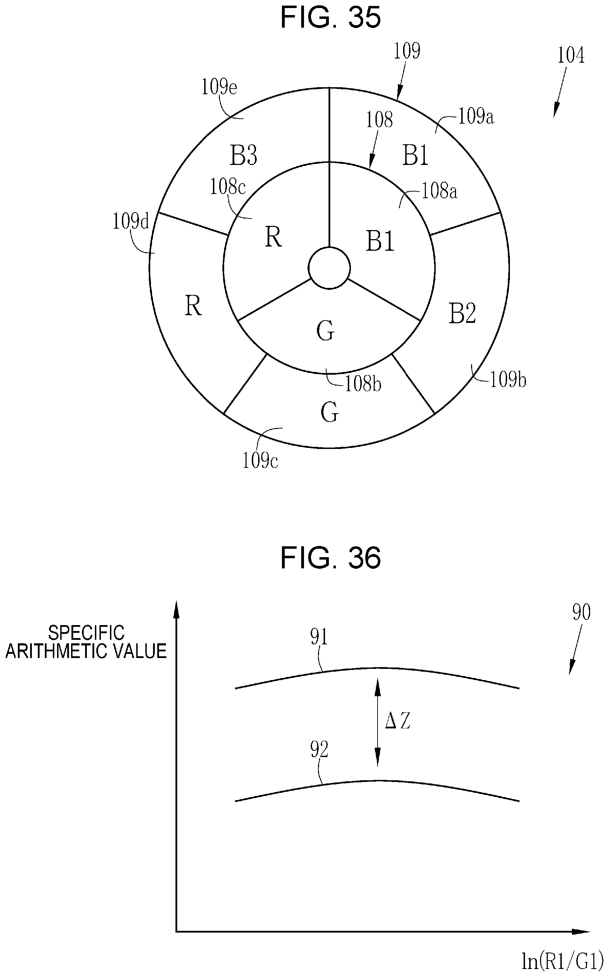

The broadband light source 102 is a xenon lamp, a white LED, or the like, and emits white light having a wavelength range ranging from blue to red. The rotary filter 104 includes an inner filter 108 disposed on the inner side and an outer filter 109 disposed on the outer side (see Fig. 35). The filter switching unit 105 is configured to move the rotary filter 104 in the radial direction. When the normal mode is set by the mode switch 12f, the filter switching unit 105 inserts the inner filter 108 of the rotary filter 104 into the optical path of white light. When the oxygen saturation mode, the correction mode, or the threshold value determination mode is set by the mode switch 12f, the filter switching unit 105 inserts the outer filter 109 of the rotary filter 104 into the optical path of white light.

As illustrated in Fig. 35, the inner filter 108 is provided with, in the circumferential direction thereof, a B1 filter 108a that transmits the violet light V and the second blue light BS of the white light, a G filter 108b that transmits the green light G of the white light, and an R filter 108c that transmits the red light R of the white light. Accordingly, in the normal mode, as the rotary filter 104 rotates, the observation target is alternately irradiated with the violet light V, the second blue light BS, the green light G, and the red light R.

The outer filter 109 is provided with, in the circumferential direction thereof, a B 1 filter 109a that transmits the first blue light BL of the white light, a B2 filter 109b that transmits the second blue light BS of the white light, a G filter 109c that transmits the green light G of the white light, an R filter 109d that transmits the red light R of the white light, and a B3 filter 109e that transmits blue-green light BG having a wavelength range B3 of the white light. Accordingly, in the oxygen saturation mode, as the rotary filter 104 rotates, the observation target is alternately irradiated with the first blue light BL, the second blue light BS, the green light G, the red light R, and the blue-green light BG.

In the endoscope system 100, in the normal mode, each time the observation target is illuminated with the violet light V, the second blue light BS, the green light G, and the red light R, imaging of the observation target is performed by the monochrome imaging sensor 106. As a result, a Bc image signal, a Gc image signal, and an Rc image signal are obtained. Then, a white-light image is generated on the basis of the image signals of the three colors in a manner similar to that in the first embodiment described above.

In the oxygen saturation mode, the correction mode, or the threshold value determination mode, by contrast, each time the observation target is illuminated with the first blue light BL, the second blue light BS, the green light G, the red light R, and the blue-green light BG, imaging of the observation target is performed by the monochrome imaging sensor 106. As a result, a B1 image signal, a B2 image signal, a G2 image signal, an R2 image signal, and a B3 image signal are obtained. The oxygen saturation mode or the correction mode is performed on the basis of the image signals of the five colors in a manner similar to that of the first embodiment. In the second embodiment, however, a signal ratio ln (B3/G2) is used instead of the signal ratio ln (B3/G3).

In the first and second embodiments described above, table correction processing for correcting the oxygen saturation calculation table 73 is performed as the correction processing related to the calculation of the oxygen saturation in the correction mode. Alternatively, calculation value correction processing for adding or subtracting a correction value obtained from the specific pigment concentration to or from the oxygen saturation calculated on the basis of the oxygen saturation calculation table 73 may be performed.

Specifically, in the calculation value correction processing, two-dimensional coordinates 90 illustrated in Fig.

36 are used to calculate a correction value to be used for correcting the oxygen saturation calculated on the basis of the oxygen saturation calculation table 73. The vertical axis of the two-dimensional coordinates represents a specific arithmetic value obtained on the basis of the B1 image signal, the G2 image signal, the R2 image signal, and the B3 image signal, and the horizontal axis thereof represents Ln (R2/G2). The specific arithmetic value is determined by Expression (A) below.

$$\text{Expression (A) } B1/G2 \times \cos\varphi - B3/G2 \times \sin\varphi$$

The two-dimensional coordinates 90 present a reference line 91 indicating the distribution of predetermined reference baseline information and an actual measurement line 92 indicating the distribution of actual measurement baseline information obtained by actual imaging of the observation target. A difference value $\Delta Z$ between the reference line 91 and the actual measurement line 92 is calculated as a correction value. In the calculation value correction processing, the correction value is added to or subtracted from the oxygen saturation calculated on the basis of the oxygen saturation calculation table 73. The reference baseline information is obtained in the absence of the specific pigment and is determined as information independent of the oxygen saturation. Specifically, a value obtained by adjusting $\varphi$ so that Expression (A) described above is kept constant even when the oxygen saturation changes is set as the reference baseline information.

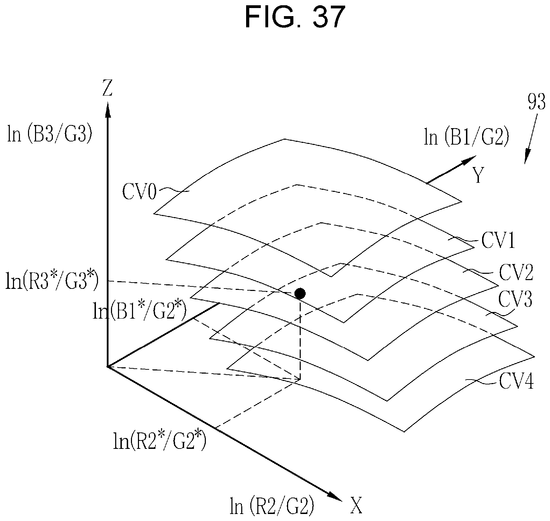

In the correction mode, instead of the correction processing, specific oxygen saturation calculation processing for calculating the oxygen saturation in accordance with the specific pigment concentration on the basis of at least the oxygen-saturation image signal and the specific pigment image signal may be performed. Specifically, three-dimensional coordinates 93 illustrated in Fig. 37 are used for the specific oxygen saturation calculation processing. In the three-dimensional coordinates 93, the X-axis is assigned the signal ratio ln (R2/G2), the Y-axis is assigned the signal ratio ln (B1/G2), and the Z-axis is assigned ln (B3/G3). Curved surfaces CV0 to CV4 represent the states of the oxygen saturations corresponding to the specific pigment concentrations "0" to "4" at the three-dimensional coordinates 93.

In the specific oxygen saturation calculation processing, at the three-dimensional coordinates 93, a value obtained by plotting on the three-dimensional coordinates 93 the signal ratios ln (R1*/G1*), ln (B2*/G1*), and ln (B3*/G3*) calculated on the basis of the B1 image signal, the G2 image signal, the R2 image signal, the B3 image signal, and the G3 image signal is calculated as the oxygen saturation. The calculated oxygen saturation is not affected by the specific pigment concentrations and is thus an accurate value.

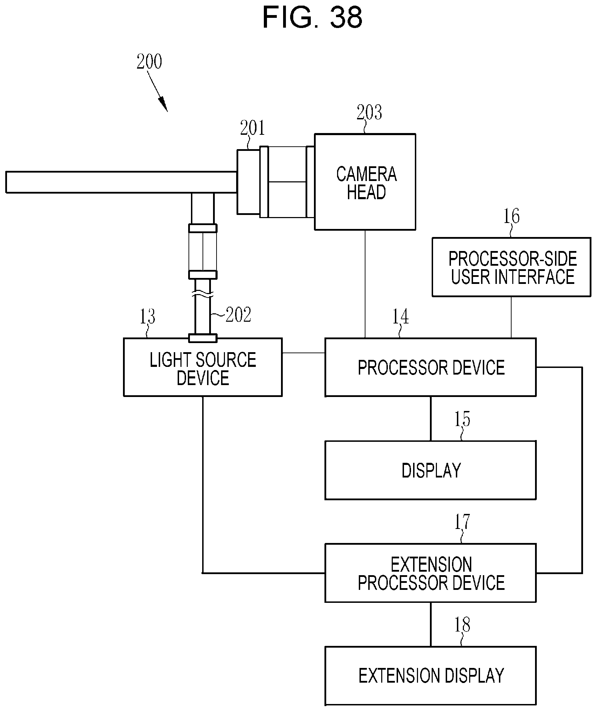

In the first and second embodiments, the endoscope 12, which is a soft endoscope for digestive-tract endoscopy, is used. Alternatively, an endoscope serving as a rigid endoscope for laparoscopic endoscopy may be used. When an endoscope that is a rigid endoscope is used, an endoscope system 200 illustrated in Fig. 38 is used. The endoscope system 200 includes an endoscope 201, a light source device 13, a processor device 14, a display 15, a processor-side user interface 16, an extension processor device 17, and an extension display 18. In the following, portions of the endoscope system 200 common to those of the first and second embodiments will not be described, and only different portions will be described.

The endoscope 201, which is used for laparoscopic surgery or the like, is formed to be rigid and elongated and is inserted into a subject. The endoscope 201 illuminates the observation target with illumination light supplied from the light source device 13 via a light guide 202. Further, the endoscope 201 receives reflected light from the observation target being illuminated with the illumination light. An imaging unit 203 is attached to the endoscope 201 and is configured to perform imaging of the observation target on the basis of reflected light guided from the endoscope 201. An image signal obtained by the imaging unit 203 through imaging is transmitted to the processor device 14.

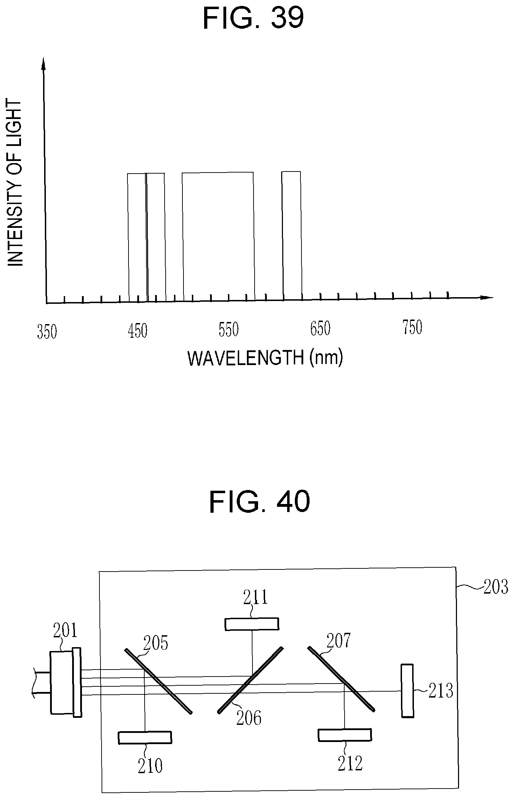

In the normal mode, the light source device 13 supplies white light including the violet light V, the second blue light BS, the green light G, and the red light R to the endoscope 201. In the oxygen saturation mode, the correction mode, or the threshold value determination mode, as illustrated in Fig. 39, the light source device 13 supplies mixed light including the first blue light BL, the second blue light BS, the green light G, and the red light R to the endoscope 12.

As illustrated in Fig. 40, the imaging unit 203 disperses white light from the endoscope 201 into light of a plurality of wavelength ranges and acquires a base-image-generation image signal to be used to generate a base image and an oxygen-saturation-calculation image signal to be used to calculate an oxygen saturation, on the basis of the dispersed light of the plurality of wavelength ranges.

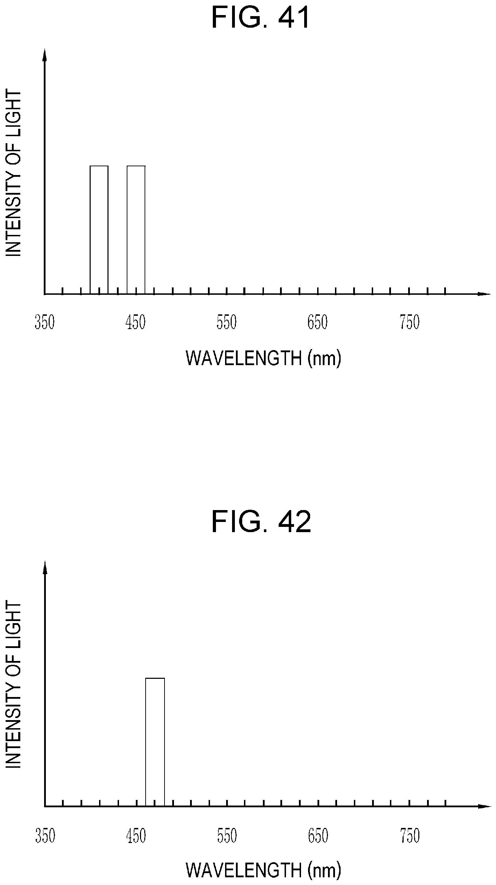

The imaging unit 203 includes dichroic mirrors 205, 206, and 207, and monochrome imaging sensors 210, 211, 212, and 213. The dichroic mirror 205 reflects, of the reflected light of the white light from the endoscope 201, the violet light V and the second blue light BS and transmits the first blue light BL, the green light G, and the red light R. As illustrated in Fig. 41, the violet light V or the second blue light BS reflected by the dichroic mirror 205 is incident on the imaging sensor 210. The imaging sensor 210 outputs a Bc image signal in response to the incidence of the violet light V and the second blue light BS in the normal mode, and outputs a B2 image signal in response to the incidence of the second blue light BS in the oxygen saturation mode, the correction mode, or the threshold value determination mode.

The dichroic mirror 206 reflects, of the light transmitted through the dichroic mirror 205, the first blue light BL and transmits the green light G and the red light R. As illustrated in Fig. 42, the first blue light BL reflected by the dichroic mirror 206 is incident on the imaging sensor 211. The imaging sensor 211 stops outputting an image signal in the normal mode, and outputs a B1 image signal in response to the incidence of the first blue light BL in the oxygen saturation mode, the correction mode, or the threshold value determination mode.

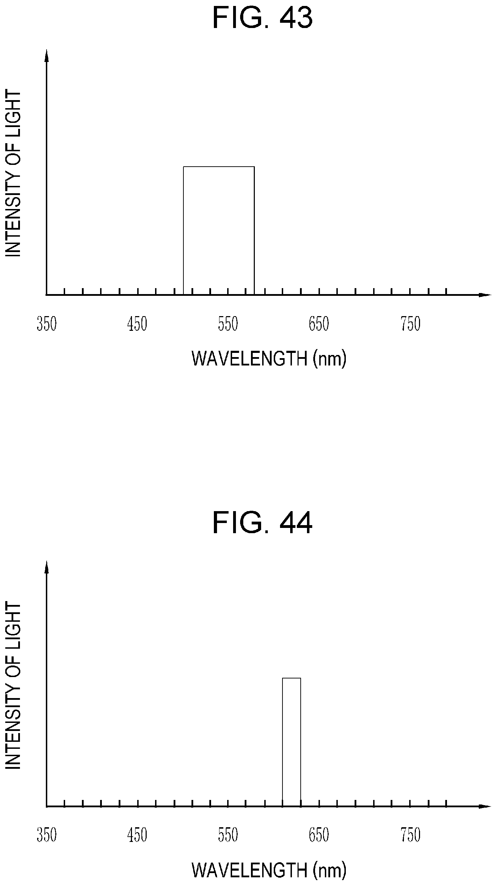

The dichroic mirror 207 reflects, of the light transmitted through the dichroic mirror 206, the green light G and transmits the red light R. As illustrated in Fig. 43, the green light G reflected by the dichroic mirror 207 is incident on the imaging sensor 212. The imaging sensor 212 outputs a Gc image signal in response to the incidence of the green light G in the normal mode, and outputs a G2 image signal in response to the incidence of the green light G in the oxygen saturation mode, the correction mode, or the threshold value determination mode.

As illustrated in Fig. 44, the red light R transmitted through the dichroic mirror 207 is incident on the imaging sensor 213. The imaging sensor 213 outputs an Rc image signal in response to the incidence of the red light R in the normal mode, and outputs an R2 image signal in response to the incidence of the red light R in the oxygen saturation mode, the correction mode, or the threshold value determination mode. As described above, in the oxygen saturation mode, the correction mode, or the threshold value determination mode, the B2 image signal, the G2 image signal, and the R2 image signal are obtained as base-image-generation image signals, and the B1 image signal, the G2 image signal, and the R2 image signal are obtained as oxygen-saturation-calculation image signals.

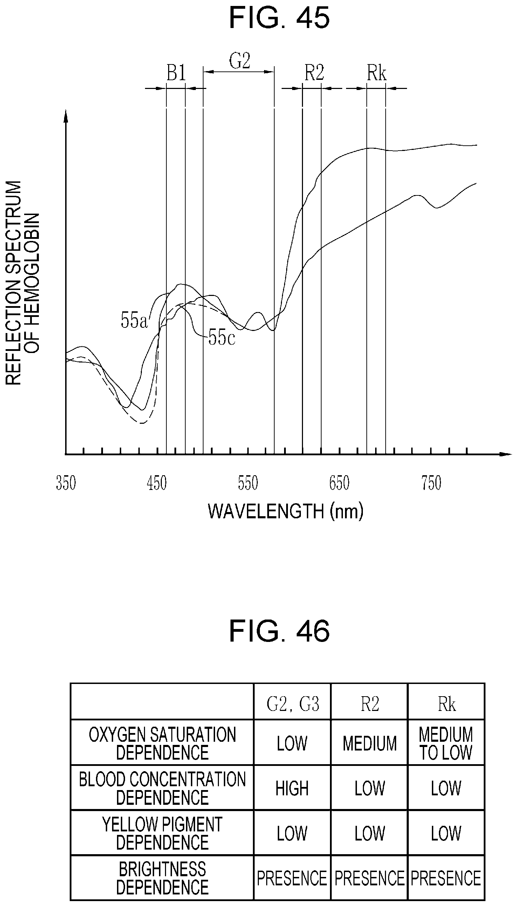

In the first and second embodiments described above, the B1 image signal, the G2 image signal, and the R2 image signal including the image information of the short-wavelength-side wavelength range B 1 in which the reflection spectrum changes in accordance with a change in the oxygen saturation of blood hemoglobin are used to calculate the oxygen saturation. Alternatively, any other image signal may be used instead of the B1 image signal. For example, as illustrated in Fig. 45, instead of the B1 image signal, an Rk image signal including image information of a long-wavelength-side wavelength range Rx in which the reflection spectrum changes in accordance with a change in the oxygen saturation of blood hemoglobin may be used. In this case, the first illumination light including the long-wavelength-side wavelength range Rx in which the reflection spectrum changes in accordance with a change in the oxygen saturation of blood hemoglobin is used. The wavelength range Rx is preferably 680 nm $\pm$ 10 nm. As illustrated in Fig. 46, the Rk image signal has "medium to low" oxygen saturation dependence, but has "low" blood concentration dependence and "low" yellow pigment dependence. Accordingly, even in a situation where the yellow pigment is present in the observation target, the oxygen saturation can be accurately calculated using only three image signals, namely, the three G2 image signals, the R2 image signal, and the Rk image signal (using only the first illumination light image and the second illumination light image).

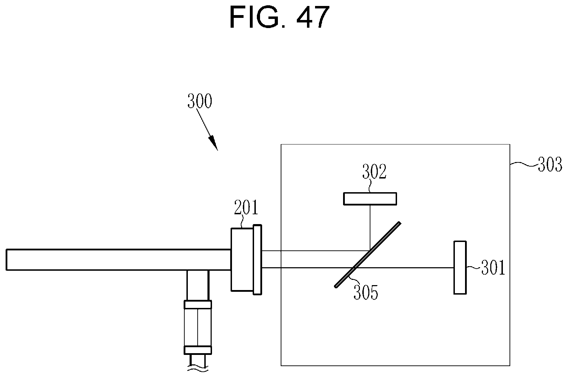

When the endoscope (see Fig. 38), which is a rigid endoscope for laparoscopic endoscopy, is used, unlike the endoscope 201 (see Fig. 43) that performs imaging of the observation target by using the four monochrome imaging sensors 210 to 213, the endoscope may be used to perform imaging of the observation target by using any other imaging method. As illustrated in Fig. 47, an endoscope 300 is a two-sensor endoscope for the abdominal cavity having one color imaging sensor 301 and one monochrome imaging sensor 302. A camera head 303 of the endoscope 300 is provided with, in addition to the color imaging sensor 301 and the monochrome imaging sensor 302, a dichroic mirror 305 that transmits part of the light incident on the camera head 303 and reflects the remaining part of the light.

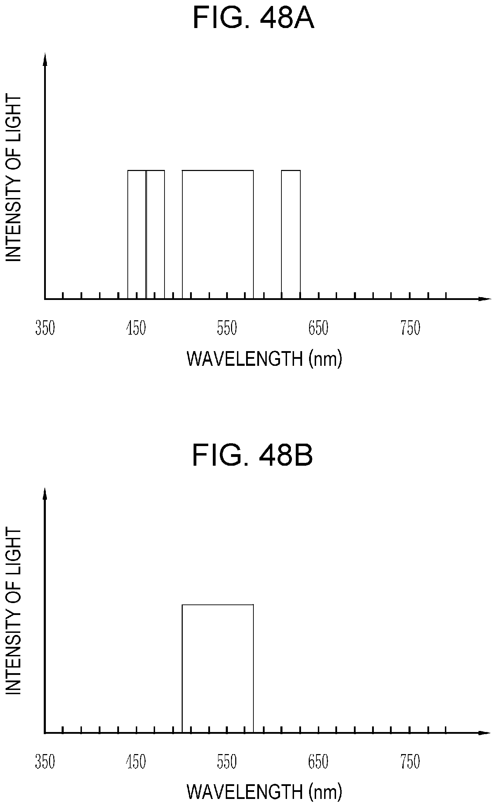

In the light emission control of the light source device 13 when the endoscope 300 is used, as illustrated in Figs. 48A and 48B, a white frame (see Fig. 48A) in which the first blue light BL, the second blue light BS, the green light G, and the red light R are simultaneously emitted and a green frame (see Fig. 48B) in which only the green light G is emitted are switched and emitted in accordance with a specific light emission pattern.

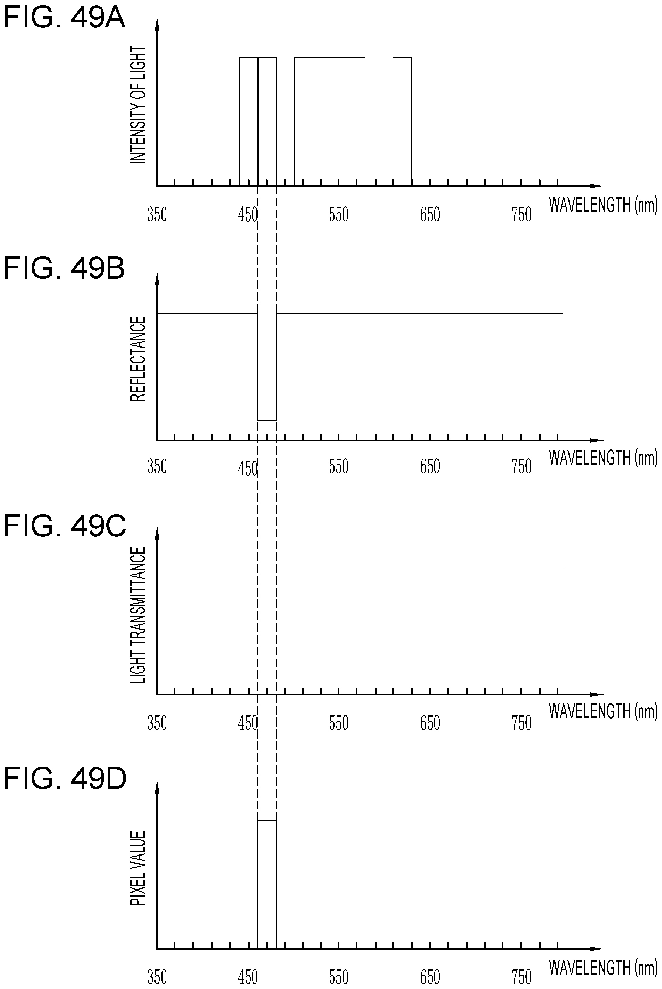

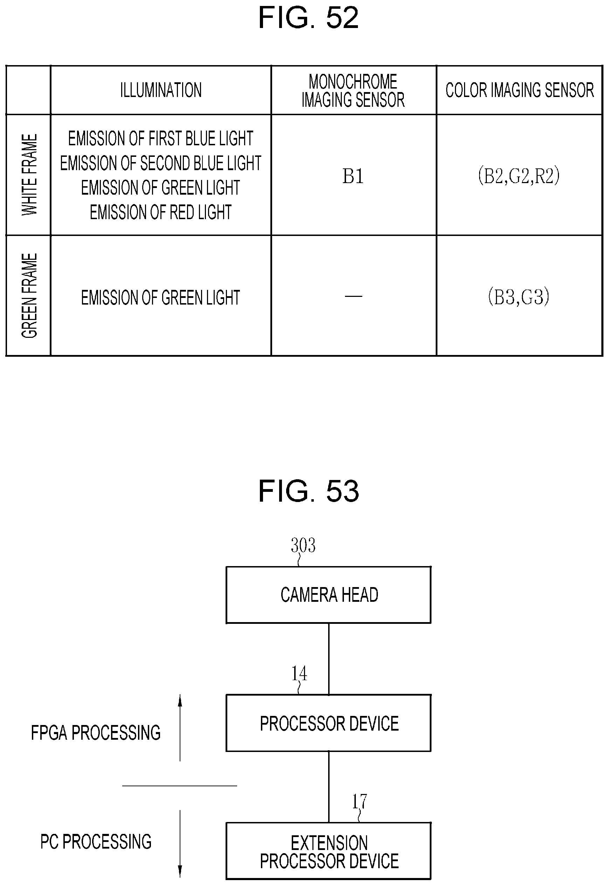

As illustrated in Figs. 49A to 49D, when the first blue light BL, the second blue light BS, the green light G, and the red light R are simultaneously emitted in the white frame (see Fig. 49A), of the light incident on the camera head 303, the first blue light BL is transmitted through the dichroic mirror 305 (see Fig. 49B), and the other light, namely, the second blue light BS, the green light G, and the red light R, is reflected by the dichroic mirror 305 (see Fig. 52B). The first blue light BL transmitted through the dichroic mirror 305 is incident on the monochrome imaging sensor 302 (see Fig. 49C). The monochrome imaging sensor 302 outputs a B1 image signal having a pixel value corresponding to the incident first blue light BL (see Fig. 49D).

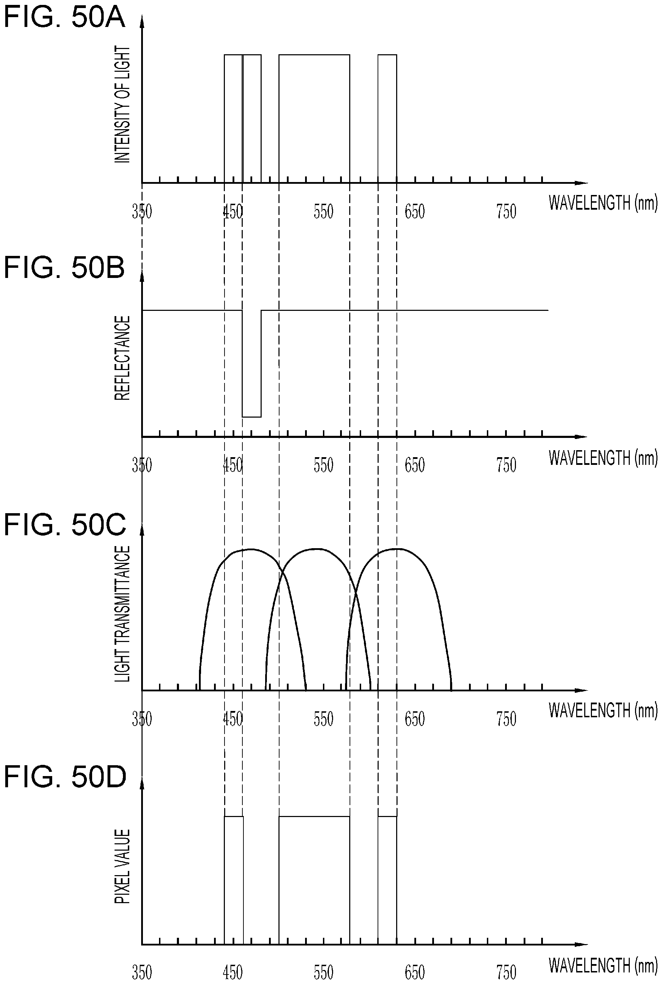

Further, as illustrated in Figs. 50A to 50D, in the white frame, the second blue light BS, the green light G, and the red light R reflected by the dichroic mirror 305 are incident on the color imaging sensor 301 (see Fig. 53C). In the color imaging sensor 301, the B pixels output a B2 image signal having a pixel value corresponding to the light transmitted through the B color filter BF out of the second blue light. The G pixels output a G2 image signal having a pixel value corresponding to the light transmitted through the G color filter GF out of the green light G. The R pixels output an R2 image signal having a pixel value corresponding to the light transmitted through the R color filter RF out of the red light R.

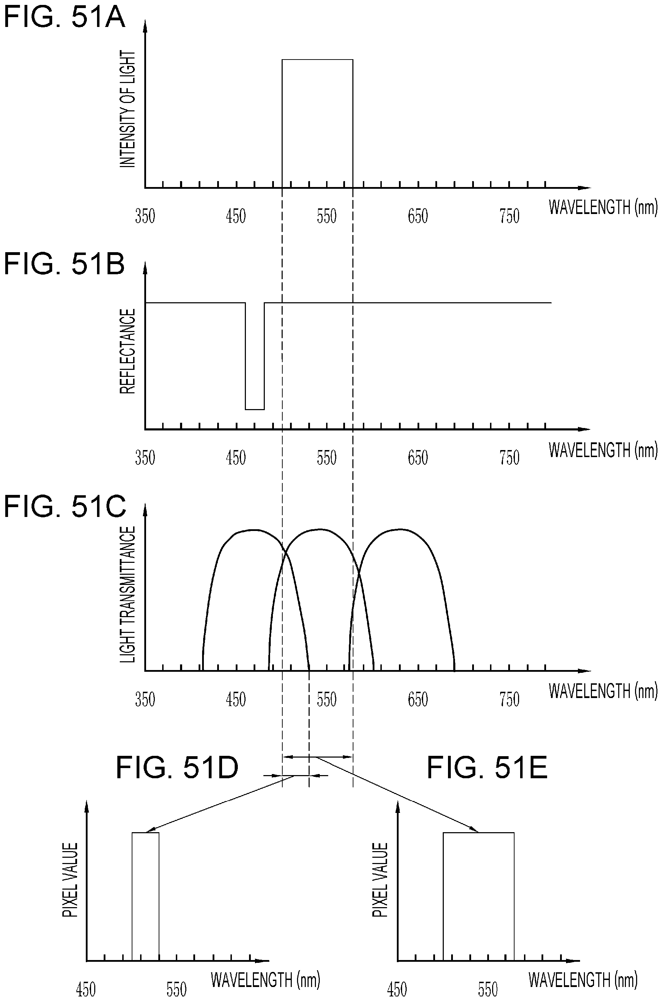

In contrast, as illustrated in Figs. 51A to 51E, when only the green light G is emitted in the green frame (see Fig. 51A), the green light G incident on the camera head 303 is reflected by the dichroic mirror 305. The green light G reflected by the dichroic mirror 305 is incident on the color imaging sensor 301. In the color imaging sensor 301, the B pixels output a B3 image signal having a pixel value corresponding to light transmitted through the B color filter BF out of the green light G. The G pixels output a G3 image signal having a pixel value corresponding to light transmitted through the G color filter GF out of the green light G. In the green frame, the image signals output from the monochrome imaging sensor 302 and the image signals output from the R pixels of the color imaging sensor 301 are not used in the subsequent processing steps.

As illustrated in Fig. 52, as described above, in a white frame, a B1 image signal is output from the monochrome imaging sensor 302, and a B2 image signal, a G2 image signal, and an R2 image signal are output from the color imaging sensor 301. The B1, B2, G2, and R2 image signals are used in the subsequent processing steps. In a green frame, by contrast, a B3 image signal and a G3 image signal are output from the color imaging sensor 301 and are used in the subsequent processing steps.

As illustrated in Fig. 53, the image signals output from the camera head 303 are sent to the processor device 14, and data on which various types of processing are performed by the processor device 14 is sent to the extension processor device 17. When the endoscope 300 is used, the processing load on the processor device 14 is taken into account, and the processes are performed in the oxygen saturation mode and the correction mode such that the processor device 14 performs low-load processing and then the extension processor device 17 performs high-load processing. Of the processes to be performed in the oxygen saturation mode and the correction mode, the processing to be performed by the processor device 14 is mainly performed by an FPGA (Field-Programmable Gate Array) and is thus referred to as FPGA processing. On the other hand, the processing to be performed by the extension processor device 17 is referred to as PC processing since the extension processor device is implemented as a PC (Personal Computer).

When the endoscope 300 is provided with an FPGA (not illustrated), the FPGA of the endoscope 300 may perform the FPGA processing. While the following describes the FPGA processing and the PC processing in the correction mode, the processes are preferably divided into the FPGA processing and the PC processing also in the oxygen saturation mode to share the processing load.

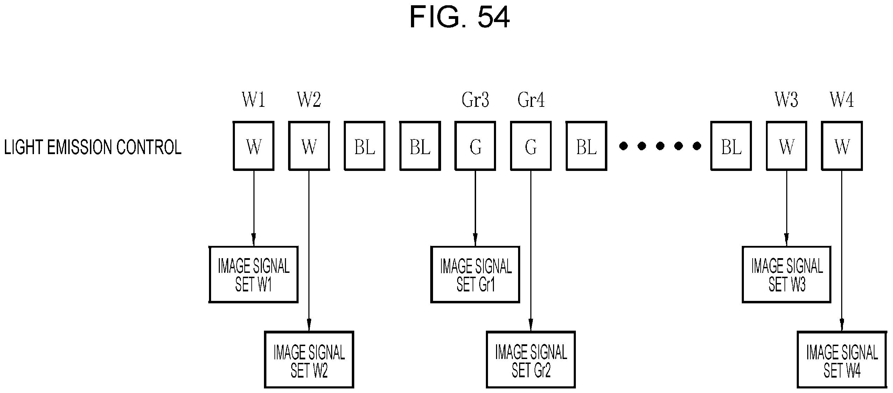

In a case where the endoscope 300 is used and light emission control is performed for a white frame W and a green frame in accordance with a specific light emission pattern, as illustrated in Fig. 54, the specific light emission pattern is such that light is emitted in two white frames W and then two blank frames BL are used in which no light is emitted from the light source device 13. Thereafter, light is emitted in two green frames G, and then two or more several (e.g., seven) blank frames are used. Thereafter, light is emitted again in two white frames W. The specific light emission pattern described above is repeatedly performed. As in the specific light emission pattern described above, light is emitted in the white frame W and the green frame Gr at least in the correction mode. In the oxygen saturation mode, light may be emitted in only the white frame W, but no light is emitted in the green frame Gr.

In the following, of the first two white frames, the first white frame is referred to as a white frame W1, and the subsequent white frame is referred to as a white frame W2 to distinguish the light emission frames in which light is emitted in accordance with a specific light emission pattern. Of the two green frames, the first green frame is referred to as a green frame Gr1, and the subsequent green frame is referred to as a green frame Gr2. Of the last two white frames, the first white frame is referred to as a white frame W3, and the subsequent white frame is referred to as a white frame W4.

The image signals for the correction mode (the B1 image signal, the B2 image signal, the G2 image signal, the R2 image signal, the B3 image signal, and the G3 image signal) obtained in the white frame W1 are referred to as an image signal set W1. Likewise, the image signals for the correction mode obtained in the white frame W2 are referred to as an image signal set W2. The image signals for the correction mode obtained in the green frame Gr1 are referred to as an image signal set Gr1. The image signals for the oxygen saturation mode and the correction mode obtained in the green frame Gr2 are referred to as an image signal set Gr2. The image signals for the correction mode obtained in the white frame W3 are referred to as an image signal set W3. The image signals for the correction mode obtained in the white frame W4 are referred to as an image signal set W4. The image signals for the threshold value determination mode or the oxygen saturation mode are image signals included in a white frame (the B1 image signal, the B2 image signal, the G2 image signal, and the R2 image signal).

The number of blank frames between the white frame W and the green frame W is desirably about two because it is only required to eliminate the light other than the green light G, whereas the number of blank frames between the green frame G and the white frame W is two or more because it is necessary to take time to stabilize the light emission state because of the start of turning on the light other than the green light G.

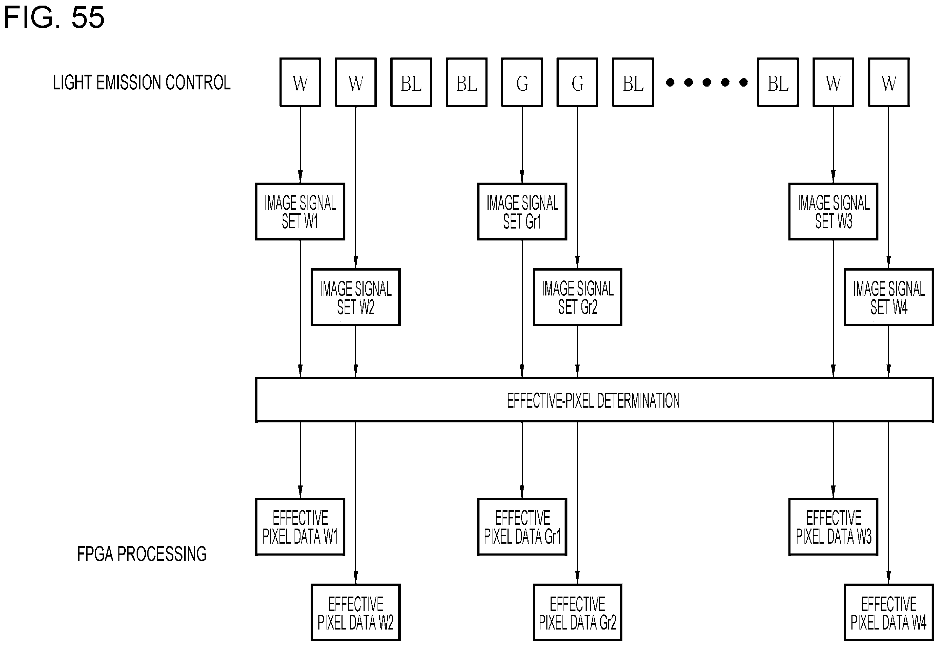

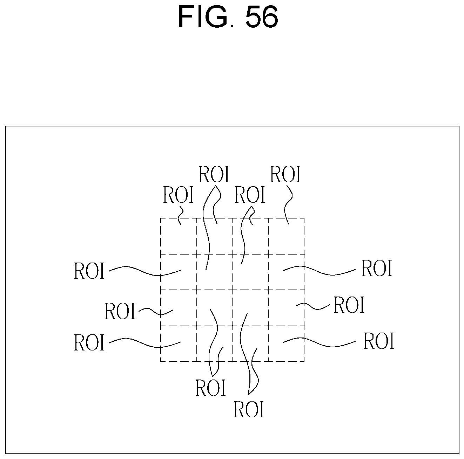

In the FPGA processing, as illustrated in Fig. 55, the pixels of all the image signals included in the image signal sets W1, W2, Gr1, Gr2, W3, and W4 are subjected to effective-pixel determination to determine whether the processing can be accurately performed in the oxygen saturation mode or the correction mode. As illustrated in Fig. 56, the effective-pixel determination is performed on the basis of pixel values in 16 regions of interest ROI provided in a center portion of an image. Specifically, for each of the pixels in the ROIs, if the pixel value falls within a range between an upper limit threshold value and a lower limit threshold value, the pixel is determined to be an effective pixel. The effective-pixel determination is performed on the pixels of all the image signals included in the image signal sets. The upper limit threshold value or the lower limit threshold value is set in advance in accordance with the sensitivity of the B pixels, the G pixels, and the R pixels of the color imaging sensor 301 or the sensitivity of the monochrome imaging sensor 302.

On the basis of the effective-pixel determination described above, the number of effective pixels, the total pixel value of the effective pixels, and the sum of squares of the pixel values of the effective pixels are calculated for each ROI. The number of effective pixels, the total pixel value of the effective pixels, and the sum of squares of the pixel values of the effective pixels for each ROI are output to the extension processor device 17 as each of pieces of effective pixel data W1, W2, Gr1, Gr2, W3, and W4. The FPGA processing is arithmetic processing using image signals of the same frame, such as effective-pixel determination, and has a lighter processing load than arithmetic processing using inter-frame image signals of different light emission frames, such as PC processing described below. The pieces of effective pixel data W1, W2, Gr1, Gr2, W3, and W4 correspond to pieces of data obtained by performing effective-pixel determination on all the image signals included in the image signal sets W1, W2, Gr1, Gr2, W3, and W4, respectively.

In the PC processing, intra-frame PC processing and inter-frame PC processing are performed on image signals of the same frame and image signals of different frames, respectively, among the pieces of effective pixel data W1, W2, Gr1, Gr2, W3, and W4. In the intra-frame PC processing, the average value of pixel values, the standard deviation value of the pixel values, and the effective pixel rate in the ROIs are calculated for all the image signals included in each piece of effective pixel data. The average value of the pixel values and the like in the ROIs, which are obtained by the intra-frame PC processing, are used in an arithmetic operation for obtaining a specific result in the oxygen saturation mode or the correction mode.

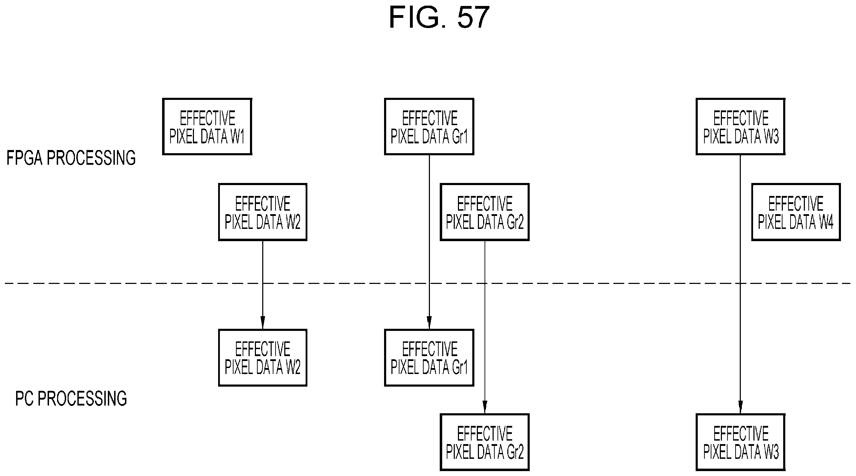

In the inter-frame PC processing, as illustrated in Fig. 57, among the pieces of effective pixel data W1, W2, Gr1, Gr2, W3, and W4 obtained in the FPGA processing, effective pixel data having a short time interval between the white frame and the green frame is used, and the other effective pixel data is not used in the inter-frame PC processing. Specifically, a pair of the effective pixel data W2 and the effective pixel data Gr1 and a pair of the effective pixel data Gr2 and the effective pixel data W3 are used in the inter-frame PC processing. The other pieces of effective pixel data W1 and W4 are not used in the inter-frame PC processing. The use of a pair of image signals having a short time interval provides accurate inter-frame PC processing without misalignment of pixels.

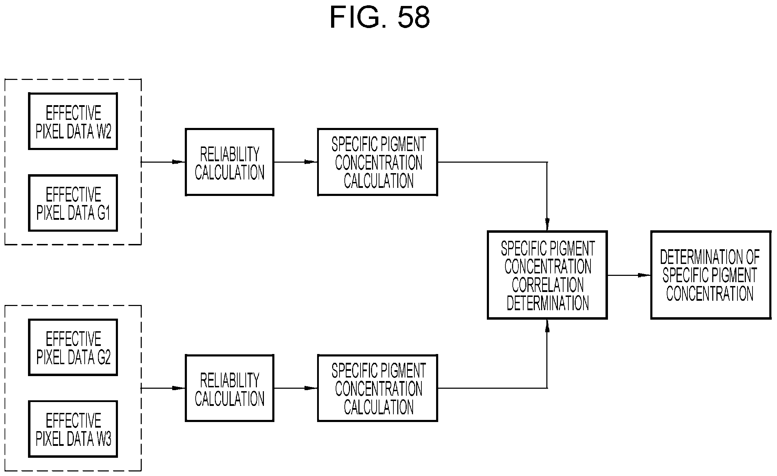

As illustrated in Fig. 58, the inter-frame PC processing using the pair of the effective pixel data W2 and the effective pixel data Gr1 involves reliability calculation and specific pigment concentration calculation, and the inter-frame PC processing using the pair of the effective pixel data Gr2 and the effective pixel data W3 also involves reliability calculation and specific pigment concentration calculation. Then, specific pigment concentration correlation determination is performed on the basis of the calculated specific pigment concentrations.

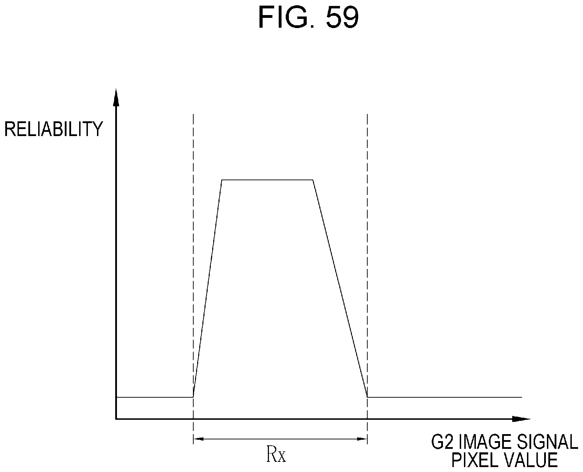

In the calculation of the reliability, the reliability is calculated for each of the 16 ROIs. In one method for calculating the reliability, for example, as illustrated in Fig. 59, the reliability for a brightness value of a G2 image signal outside the certain range Rx is preferably set to be lower than the reliability for a brightness value of a G2 image signal within the certain range Rx. In the case of the pair of the effective pixel data W2 and the effective pixel data Gr1, a total of 32 reliabilities are calculated by reliability calculation of a G2 image signal included in each piece of effective pixel data for each ROI. Likewise, in the pair of the effective pixel data Gr2 and the effective pixel data W3, a total of 32 reliabilities are calculated. When the reliability is calculated, for example, if a ROI having low reliability is present or if the average reliability value of the ROIs is less than a predetermined value, error determination is performed for the reliability. The result of the error determination for the reliability is displayed on the extension display 18 or the like to provide a notification to the user.

In the specific pigment concentration calculation, a specific pigment concentration is calculated for each of the 16 ROIs. The method for calculating the specific pigment concentration is similar to the calculation method performed by the specific pigment concentration calculation unit 62 described above. For example, the specific pigment concentration calculation table 62a is referred to by using the B 1 image signal, the G2 image signal, the R2 image signal, the B3 image signal, and the G3 image signal included in the effective pixel data W2 and the effective pixel data Gr1, and a specific pigment concentration corresponding to the signal ratios ln (B1/G2), ln (G2/R2), and ln (B3/G3) is calculated. As a result, a total of 16 specific pigment concentrations PG1 are calculated for the respective ROIs. Also in the case of the pair of the effective pixel data Gr2 and the effective pixel data W3, a total of 16 specific pigment concentrations PG2 are calculated for the respective ROIs in a similar manner.

When the specific pigment concentrations PG1 and the specific pigment concentrations PG2 are calculated, correlation values between the specific pigment concentrations PG1 and the specific pigment concentrations PG2 are calculated for the respective ROIs. The correlation values are preferably calculated for the respective ROIs at the same position. If a certain number or more of ROIs having correlation values lower than a predetermined value are present, it is determined that a motion has occurred between the frames, and error determination for the motion is performed. The result of the error determination for the motion is notified to the user by, for example, being displayed on the extension display 18.

If no error is present in the error determination for the motion, one specific pigment concentration is calculated from among the total of 32 specific pigment concentrations PG1 and specific pigment concentrations PG2 by using a specific estimation method (e.g., a robust estimation method). The calculated specific pigment concentration is used in the correction processing for the correction mode. The correction processing for the correction mode is similar to that described above, such as table correction processing.

In the embodiments described above, the hardware structures of processing units that perform various types of processing, such as the oxygen saturation image generation unit 60, the threshold value determination unit 61, the specific pigment concentration calculation unit 62, the table correction unit 63, a mode switching unit 64, a display style control unit 65, a reliability calculation unit 66, a first correction determination unit 67, a second correction determination unit 68, a determination notification unit 69, the base image generation unit 70, the arithmetic value calculation unit 71, the oxygen saturation calculation unit 72, and the color tone adjustment unit 74, are various processors described below. The various processors include a CPU (Central Processing Unit), which is a general-purpose processor executing software (program) to function as various processing units, a GPU (Graphical Processing Unit), a programmable logic device (PLD) such as an FPGA (Field Programmable Gate Array), which is a processor whose circuit configuration is changeable after manufacturing, a dedicated electric circuit, which is a processor having a circuit configuration specifically designed to execute various types of processing, and so on.

A single processing unit may be configured as one of these various processors or as a combination of two or more processors of the same type or different types (such as a plurality of FPGAs, a combination of a CPU and an FPGA, or a combination of a CPU and a GPU, for example). Alternatively, a plurality of processing units may be configured as a single processor. Examples of configuring a plurality of processing units as a single processor include, first, a form in which, as typified by a computer such as a client or a server, the single processor is configured as a combination of one or more CPUs and software and the processor functions as the plurality of processing units. The examples include, second, a form in which, as typified by a system on chip (SoC) or the like, a processor is used in which the functions of the entire system including the plurality of processing units are implemented as one IC (Integrated Circuit) chip. As described above, the various processing units are configured by using one or more of the various processors described above as a hardware structure.

More specifically, the hardware structure of these various processors is an electric circuit (circuitry) in which circuit elements such as semiconductor elements are combined. The hardware structure of a storage unit is a storage device such as an HDD (hard disc drive) or an SSD (solid state drive).

Reference Signs List

| | |
|---|---|
| 10, 100 | endoscope system |
| 12 | endoscope |
| 12a | insertion section |
| 12b | operation section |
| 12c | bending part |
| 12d | tip part |
| 12e | angle knob |
| 12f | mode switch |
| 12g | still-image acquisition instruction switch |
| 12h | still-image acquisition instruction switch |
| 12i | zoom operation unit |
| 12j | forceps port |
| 13 | light source device |
| 14 | processor device |
| 15 | display |
| 16 | processor-side user interface |
| 17 | extension processor device |
| 18 | extension display |
| 19 | scope-side user interface |
| 20 | light source unit |
| 20a | V-LED |
| 20b | BS-LED |
| 20c | BL-ELD |
| 20d | G-LED |
| 20e | R-LED |
| 21 | light-source processor |

| | |
|---|---|
| 23 | optical path coupling unit |
| 25 | light guide |
| 30 | illumination optical system |
| 31 | imaging optical system |
| 32 | illumination lens |
| 35 | objective lens |
| 36, 106 | imaging sensor |
| 37 | imaging processor |
| 40 | CDS/AGD circuit |
| 41 | A/D converter |
| 45 | DSP |
| 50 | image processing unit |
| 52 | display control unit |
| 53 | central control unit |
| 55a, b, c | curve |
| 60 | oxygen saturation image generation unit |
| 61 | threshold value determination unit |
| 62 | specific pigment concentration calculation unit |
| 62a | specific pigment concentration calculation table |
| 63 | table correction unit |
| 70 | base image generation unit |
| 71 | arithmetic value calculation unit |
| 72 | oxygen saturation calculation unit |
| 73 | oxygen saturation calculation table |
| 74 | color tone adjustment unit |
| 80 | user operation acceptance unit |
| 81 | threshold value calculation unit |
| 83 | threshold value calculation region |
| 84 | observation image display region |
| 86 | threshold value calculation memory |
| 90 | two-dimensional coordinate |
| 91 | reference line |
| 92 | actual measurement line |
| 93 | three-dimensional coordinate |
| 102 | broadband light source |
| 104 | rotary filter |
| 105 | filter switching unit |
| 108 | inner filter |
| 108a | B1 filter |
| 108b | G filter |
| 108c | R filter |
| 109 | outer filter |
| 109a | B1 filter |
| 109b | B2 filter |
| 109c | G filter |
| 109d | R filter |
| 109e | B3 filter |
| 200 | endoscope system |
| 201 | endoscope |
| 202 | light guide |
| 203 | imaging unit |
| 205 to 207 | dichroic mirror |
| 210 to 214 | imaging sensor |
| 300 | endoscope |
| 301 | color imaging sensor |
| 302 | monochrome imaging sensor |
| 303 | camera head |
| 305 | dichroic mirror |

| AR0 | to AR4 region |
| DFX, DFY | definition line |
| BF | B color filter |
| GD | operational guidance |
| GF | G color filter |
| MS0 | message |
| RF | R color filter |
| Rx | long-wavelength-side wavelength range |
| CV0 to CV4 | curved surface |
| EL, ELL, ELH | contour |

**Claims**

1. A processor device comprising:
   a processor configured to:

   generate a base image;
   generate an oxygen saturation image in an oxygen saturation mode, the oxygen saturation image including a high-oxygen-saturation region and a low-oxygen-saturation region, the high-oxygen-saturation region being a region in which an oxygen saturation exceeds a threshold value and in which a color tone of the base image is controlled by a first color tone control method, the low-oxygen-saturation region being a region in which the oxygen saturation is less than or equal to the threshold value and in which the color tone of the base image is controlled by a second color tone control method different from the first color tone control method; and
   in a threshold value determination mode for determining the threshold value, display a threshold value calculation region on a display and calculate the threshold value based on at least oxygen saturations in the threshold value calculation region, the oxygen saturations in the threshold value calculation region being calculated in accordance with a threshold value calculation operation.

2. The processor device according to claim 1, wherein the processor is configured to:
   calculate the oxygen saturations in the threshold value calculation region at a timing at which the threshold value calculation operation is performed, and calculate the threshold value based on a representative value of the oxygen saturations in the threshold value calculation region.

3. The processor device according to claim 2, wherein the threshold value is calculated based on the representative value of the oxygen saturations in the threshold value calculation region and a correction oxygen saturation.

4. The processor device according to claim 2 or 3, wherein the threshold value calculation region includes a normal site.

5. The processor device according to claim 1, wherein the processor is configured to:
   in a case of accepting the threshold value calculation operation performed a plurality of times and accepting a confirmation operation performed after the threshold value calculation operation is performed the plurality of times, perform first processing and second processing, the first processing being for calculating the oxygen saturations in the threshold value calculation region as threshold-value-calculation oxygen saturations at a timing at which the threshold value calculation operation is performed, the second processing being for calculating the threshold value in response to the confirmation operation being performed, the threshold value being calculated based on the threshold-value-calculation oxygen saturations calculated in each threshold value calculation operation.

6. The processor device according to claim 5, wherein the threshold value is calculated based on a plurality-of-operation representative value and a correction oxygen saturation, the plurality-of-operation representative value being obtained from representative values of the threshold-value-calculation oxygen saturations calculated in respective threshold value calculation operations.

7. The processor device according to claim 5 or 6, wherein the threshold value calculation region includes a normal site or a hypoxic site.

8. The processor device according to claim 3 or 6, wherein the correction oxygen saturation is determined based on dynamics of an observation target or individual differences between patients.

9. The processor device according to any one of claims 1 to 7, wherein

when the first color tone control method is a method for maintaining the color tone of the base image regardless of the oxygen saturation, the second color tone control method is a method for changing the color tone of the base image in accordance with the oxygen saturation, and
when the first color tone control method is a method for changing the color tone of the base image in accordance with the oxygen saturation, the second color tone control method is a method for maintaining the color tone of the base image regardless of the oxygen saturation.

10. An endoscope system comprising:

the processor device according to any one of claims 1 to 9; and
a light source device that emits first illumination light, second illumination light, and third illumination light in a specific range, the first illumination light including a short-wavelength-side wavelength range in which an absorption coefficient changes in accordance with a change in oxygen saturation of blood hemoglobin, wherein the processor is configured to:

generate the base image based on a second illumination light image that is based on the second illumination light; and
calculate the oxygen saturation based on a first illumination light image that is based on the first illumination light, the second illumination light image, and a third illumination light image that is based on the third illumination light.

11. An endoscope system comprising:

the processor device according to any one of claims 1 to 9; and
a light source device that emits first illumination light and second illumination light, the first illumination light including a long-wavelength-side wavelength range in which an absorption coefficient changes in accordance with a change in oxygen saturation of blood hemoglobin, wherein the processor is configured to:

generate the base image based on a second illumination light image that is based on the second illumination light; and
calculate the oxygen saturation based on a first illumination light image and the second illumination light image, the first illumination light image being based on the first illumination light.

12. An endoscope system comprising:

the processor device according to any one of claims 1 to 9;
a light source device that supplies white light to an endoscope; and
an imaging unit that is to be attached to the endoscope and that disperses the white light from the endoscope into light of a plurality of wavelength ranges and acquires a base-image-generation image signal to be used to generate the base image and an oxygen-saturation-calculation image signal to be used to calculate the oxygen saturation, based on the dispersed light of the plurality of wavelength ranges.

13. A method for operating a processor device, the method comprising the steps of, by a processor:

generating a base image;
generating an oxygen saturation image in an oxygen saturation mode, the oxygen saturation image including a high-oxygen-saturation region and a low-oxygen-saturation region, the high-oxygen-saturation region being a region in which an oxygen saturation exceeds a threshold value and in which a color tone of the base image is controlled by a first color tone control method, the low-oxygen-saturation region being a region in which the oxygen saturation is less than or equal to the threshold value and in which the color tone of the base image is controlled by a second color tone control method different from the first color tone control method; and
in a threshold value determination mode for determining the threshold value, displaying a threshold value calculation region on a display and calculating the threshold value based on at least oxygen saturations in the threshold value calculation region, the oxygen saturations in the threshold value calculation region being calculated in accordance with a threshold value calculation operation.

# FIG. 1

# FIG. 2

15

WHITE-LIGHT
IMAGE

18

# FIG. 3

15

WHITE-LIGHT-
EQUIVALENT IMAGE

18

OXYGEN
SATURATION IMAGE

# FIG. 4

18

MS0

Please perform correction processing.

# FIG. 5A

18

INTERNAL-DIGESTIVE-
TRACT OXYGEN
SATURATION IMAGE

# FIG. 5B

18

SEROSA-SIDE OXYGEN
SATURATION IMAGE

FIG. 6

# FIG. 7

FIG. 8A

FIG. 8B

FIG. 8C

# FIG. 9

# FIG. 10

| ILLUMINATION | IMAGE SIGNAL |
|---|---|
| EMISSION OF VIOLET LIGHT<br>EMISSION OF SECOND BLUE LIGHT<br>EMISSION OF GREEN LIGHT<br>EMISSION OF RED LIGHT | $(Bc, Gc, Rc)$ |

# FIG. 11

| | ILLUMINATION | IMAGE SIGNAL |
|---|---|---|
| FIRST FRAME | EMISSION OF FIRST BLUE LIGHT<br>EMISSION OF GREEN LIGHT<br>EMISSION OF RED LIGHT | $(B1, G1, R1)$ |
| SECOND FRAME | EMISSION OF SECOND BLUE LIGHT<br>EMISSION OF GREEN LIGHT<br>EMISSION OF RED LIGHT | $(B2, G2, R2)$ |
| THIRD FRAME | EMISSION OF GREEN LIGHT | $(B3, G3, R3)$ |

# FIG. 12

| LIGHT EMISSION CONTROL | 1stF | 2ndF | 3rdF | 2ndF | 1stF |
|---|---|---|---|---|---|

DISPLAYED ON DISPLAY

| WHITE-LIGHT-EQUIVALENT IMAGE | WHITE-LIGHT-EQUIVALENT IMAGE |
|---|---|

DISPLAYED ON EXTENSION DISPLAY

| OXYGEN SATURATION IMAGE | OXYGEN SATURATION IMAGE |
|---|---|

# FIG. 13

# FIG. 14

# FIG. 15

|  | B1 | G2 | R2 |
|---|---|---|---|
| OXYGEN SATURATION DEPENDENCE | HIGH | LOW | MEDIUM |
| BLOOD CONCENTRATION DEPENDENCE | MEDIUM | HIGH | LOW |
| BRIGHTNESS DEPENDENCE | PRESENCE | PRESENCE | PRESENCE |

# FIG. 16

# FIG. 17

|  | X | Y |
|---|---|---|
| OXYGEN SATURATION DEPENDENCE | MEDIUM | HIGH |
| BLOOD CONCENTRATION DEPENDENCE | HIGH | MEDIUM |
| BRIGHTNESS DEPENDENCE | ABSENCE | ABSENCE |

# FIG. 18

|  | B1 | G2 | R2 |
|---|---|---|---|
| OXYGEN SATURATION DEPENDENCE | HIGH | LOW | MEDIUM |
| BLOOD CONCENTRATION DEPENDENCE | MEDIUM | HIGH | LOW |
| YELLOW PIGMENT DEPENDENCE | HIGH | LOW TO MEDIUM | LOW |
| BRIGHTNESS DEPENDENCE | PRESENCE | PRESENCE | PRESENCE |

# FIG. 19

# FIG. 20

| | B1 | B3 | G2, G3 | R2 | B2 |
|---|---|---|---|---|---|
| OXYGEN SATURATION DEPENDENCE | HIGH | LOW | LOW | MEDIUM | LOW |
| BLOOD CONCENTRATION DEPENDENCE | MEDIUM | HIGH | HIGH | LOW | HIGH |
| YELLOW PIGMENT DEPENDENCE | HIGH | MEDIUM | LOW TO MEDIUM | LOW | HIGH |
| BRIGHTNESS DEPENDENCE | PRESENCE | PRESENCE | PRESENCE | PRESENCE | PRESENCE |

# FIG. 21

FIG. 22A

FIG. 22B

# FIG. 23

|  | X | Y | Z |
|---|---|---|---|
| OXYGEN SATURATION DEPENDENCE | MEDIUM | HIGH | LOW TO MEDIUM |
| BLOOD CONCENTRATION DEPENDENCE | HIGH | MEDIUM | LOW TO MEDIUM |
| YELLOW PIGMENT DEPENDENCE | LOW TO MEDIUM | HIGH | MEDIUM |
| BRIGHTNESS DEPENDENCE | ABSENCE | ABSENCE | ABSENCE |

# FIG. 24

EXTENSION PROCESSOR DEVICE ~17

**OXYGEN SATURATION IMAGE GENERATION UNIT** ~60

- BASE IMAGE GENERATION UNIT ~70
- ARITHMETIC VALUE CALCULATION UNIT ~71
- OXYGEN SATURATION CALCULATION UNIT ~72
  - OXYGEN SATURATION CALCULATION TABLE ~73
- COLOR TONE ADJUSTMENT UNIT ~74

THRESHOLD VALUE DETERMINATION UNIT ~61

**SPECIFIC PIGMENT CONCENTRATION CALCULATION UNIT** ~62

- SPECIFIC PIGMENT CONCENTRATION CALCULATION TABLE ~62a

TABLE CORRECTION UNIT ~63

EP 4 445 827 A1

# FIG. 25

# FIG. 26

# FIG. 27

# FIG. 28

## FIG. 29

THRESHOLD VALUE DETERMINATION UNIT ~62

USER OPERATION ACCEPTANCE UNIT ~80

THRESHOLD VALUE CALCULATION UNIT ~81

## FIG. 30

18

83

84

# FIG. 31

START

SWITCH TO THRESHOLD VALUE DETERMINATION MODE

DISPLAY THRESHOLD VALUE CALCULATION REGION

PLACE NORMAL REGION WITHIN THRESHOLD VALUE CALCULATION REGION

EXECUTE THRESHOLD VALUE CALCULATION OPERATION

CALCULATE THRESHOLD VALUE

SWITCH TO OXYGEN SATURATION MODE

DISPLAY OXYGEN SATURATION IMAGE

END

# FIG. 32

THRESHOLD VALUE DETERMINATION UNIT ~62

USER OPERATION ACCEPTANCE UNIT ~80

THRESHOLD VALUE CALCULATION UNIT ~81

THRESHOLD VALUE CALCULATION MEMORY ~86

# FIG. 33

START

SWITCH TO THRESHOLD VALUE
DETERMINATION MODE

DISPLAY THRESHOLD VALUE
CALCULATION REGION

PLACE FIRST REGION WITHIN
THRESHOLD VALUE CALCULATION REGION

EXECUTE THRESHOLD VALUE
CALCULATION OPERATION

PLACE SECOND REGION WITHIN
THRESHOLD VALUE CALCULATION REGION

EXECUTE THRESHOLD VALUE
CALCULATION OPERATION

COMPLETION OF CALCULATION OF
THRESHOLD-VALUE-CALCULATION OXYGEN SATURATIONS
FOR PLURALITY OF TARGET REGIONS?

N → CALCULATE THRESHOLD-VALUE-
CALCULATION OXYGEN SATURATIONS
FOR OTHER TARGET REGIONS

Y

CALCULATE THRESHOLD VALUE

SWITCH TO OXYGEN SATURATION MODE

DISPLAY OXYGEN SATURATION IMAGE

END

# FIG. 34

100

12

14

IMAGING PROCESSOR ~37

31

35 OBJECTIVE LENS

106 IMAGING SENSOR

40 CDS/AGC CIRCUIT

41 A/D CONVERTER

53 CENTRAL CONTROL UNIT

45 DSP

50 IMAGE PROCESSING UNIT

52 DISPLAY CONTROL UNIT

15 DISPLAY

51 IMAGE COMMUNICATION UNIT

30

32 ILLUMINATION LENS

25 LIGHT GUIDE

104 ROTARY FILTER

102 BROADBAND LIGHT SOURCE

105 FILTER SWITCHING UNIT

17 EXTENSION PROCESSOR DEVICE

18 EXTENSION DISPLAY

13

EP 4 445 827 A1

# FIG. 35

# FIG. 36

# FIG. 37

# FIG. 38

# FIG. 39

# FIG. 40

# FIG. 41

INTENSITY OF LIGHT

350    450    550    650    750

WAVELENGTH (nm)

# FIG. 42

INTENSITY OF LIGHT

350    450    550    650    750

WAVELENGTH (nm)

# FIG. 43

INTENSITY OF LIGHT

WAVELENGTH (nm)

350    450    550    650    750

# FIG. 44

INTENSITY OF LIGHT

WAVELENGTH (nm)

350    450    550    650    750

# FIG. 45

# FIG. 46

|  | G2, G3 | R2 | Rk |
|---|---|---|---|
| OXYGEN SATURATION DEPENDENCE | LOW | MEDIUM | MEDIUM TO LOW |
| BLOOD CONCENTRATION DEPENDENCE | HIGH | LOW | LOW |
| YELLOW PIGMENT DEPENDENCE | LOW | LOW | LOW |
| BRIGHTNESS DEPENDENCE | PRESENCE | PRESENCE | PRESENCE |

# FIG. 47

# FIG. 48A

# FIG. 48B

## FIG. 49A

INTENSITY OF LIGHT

350    450    550    650    750    WAVELENGTH (nm)

## FIG. 49B

REFLECTANCE

350    450    550    650    750    WAVELENGTH (nm)

## FIG. 49C

LIGHT TRANSMITTANCE

350    450    550    650    750    WAVELENGTH (nm)

## FIG. 49D

PIXEL VALUE

350    450    550    650    750    WAVELENGTH (nm)

FIG. 50A

FIG. 50B

FIG. 50C

FIG. 50D

FIG. 51A

FIG. 51B

FIG. 51C

FIG. 51D

FIG. 51E

# FIG. 52

|  | ILLUMINATION | MONOCHROME IMAGING SENSOR | COLOR IMAGING SENSOR |
|---|---|---|---|
| WHITE FRAME | EMISSION OF FIRST BLUE LIGHT EMISSION OF SECOND BLUE LIGHT EMISSION OF GREEN LIGHT EMISSION OF RED LIGHT | B1 | (B2,G2,R2) |
| GREEN FRAME | EMISSION OF GREEN LIGHT | — | (B3,G3) |

# FIG. 53

# FIG. 54

LIGHT EMISSION CONTROL

W1 | W2 → W | W | BL | BL | Gr3 | Gr4 → G | G | BL ••••• BL | W3 | W4 → W | W

IMAGE SIGNAL SET W1

IMAGE SIGNAL SET W2

IMAGE SIGNAL SET Gr1

IMAGE SIGNAL SET Gr2

IMAGE SIGNAL SET W3

IMAGE SIGNAL SET W4

EP 4 445 827 A1

# FIG. 55

EP 4 445 827 A1

FIG. 56

# FIG. 57

FPGA PROCESSING

| EFFECTIVE PIXEL DATA W1 | EFFECTIVE PIXEL DATA Gr1 | EFFECTIVE PIXEL DATA W3 |

EFFECTIVE PIXEL DATA W2

EFFECTIVE PIXEL DATA Gr2

EFFECTIVE PIXEL DATA W4

EFFECTIVE PIXEL DATA W2

EFFECTIVE PIXEL DATA Gr1

PC PROCESSING

EFFECTIVE PIXEL DATA Gr2

EFFECTIVE PIXEL DATA W3

EP 4 445 827 A1

# FIG. 58

# FIG. 59

RELIABILITY

Rx

G2 IMAGE SIGNAL
PIXEL VALUE

## INTERNATIONAL SEARCH REPORT

| International application No. |
|---|
| **PCT/JP2022/042708** |

| A. | CLASSIFICATION OF SUBJECT MATTER |
|---|---|

*A61B 1/045*(2006.01)i; *A61B 1/00*(2006.01)i
FI:   A61B1/045 617; A61B1/00 513; A61B1/045 640

According to International Patent Classification (IPC) or to both national classification and IPC

| B. | FIELDS SEARCHED |
|---|---|

Minimum documentation searched (classification system followed by classification symbols)

A61B1/045; A61B1/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | JP 2012-139482 A (FUJIFILM CORP.) 26 July 2012 (2012-07-26)<br>paragraphs [0020]-[0083], fig. 1-23 | 1-13 |
| Y | WO 2019/172231 A1 (FUJIFILM CORP.) 12 September 2019 (2019-09-12)<br>paragraphs [0025]-[0074], fig. 1-20 | 1-13 |
| Y | WO 2020/235502 A1 (FUJIFILM CORP.) 26 November 2020 (2020-11-26)<br>paragraphs [0021]-[0066], fig. 1-7 | 1-13 |
| Y | WO 2020/235503 A1 (FUJIFILM CORP.) 26 November 2020 (2020-11-26)<br>paragraphs [0027]-[0120], fig. 1-28 | 5-12 |

☐ Further documents are listed in the continuation of Box C.　　☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **07 December 2022** | **24 January 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

International application No.

**PCT/JP2022/042708**

| Patent document cited in search report | | | Publication date (day/month/year) | Patent family member(s) | | | Publication date (day/month/year) |
|---|---|---|---|---|---|---|---|
| JP | 2012-139482 | A | 26 July 2012 | US paragraphs [0056]-[0124], fig. 1-23 EP | 2012/0157768 2465431 | A1 A1 | |
| WO | 2019/172231 | A1 | 12 September 2019 | US paragraphs [0066]-[0117], fig. 1-20 | 2020/0402235 | A1 | |
| WO | 2020/235502 | A1 | 26 November 2020 | US paragraphs [0038]-[0083], fig. 1-7 | 2022/0076458 | A1 | |
| WO | 2020/235503 | A1 | 26 November 2020 | US paragraphs [0053]-[0147], fig. 1-28 EP CN | 2022/0076400 3973845 113905653 | A1 A1 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012139482 A